# EUROPÄISCHE PATENTSCHRIFT

(11) **EP 0 843 667 B1**
(45) Veröffentlichungstag und Bekanntmachung des Hinweises auf die Patenterteilung: **17.10.2001**
(21) Anmeldenummer: 96926381.3
(22) Anmeldetag: 22.07.1996
(51) Int. Cl.: C07D 239/54, A01N 43/54

(54) **SUBSTITUIERTE PHENYLURACILE**
SUBSTITUTED PHENYLURACILS
PHENYLURACILES SUBSTITUES

(30) Priorität: 01.08.1995 DE 19528186
(43) Veröffentlichungstag der Anmeldung: 27.05.1998
(73) Patentinhaber: BAYER AG, 51368 Leverkusen (DE)
(72) Erfinder: ANDREE, Roland, D-40764 Langenfeld (DE); DREWES, Mark, Wilhelm, D-40764 Langenfeld (DE); DOLLINGER, Markus, D-51381 Leverkusen (DE); SANTEL, Hans-Joachim, D-51371 Leverkusen (DE)
(86) Internationale Anmeldenummer: EP9603223
(87) Internationale Veröffentlichungsnummer: WO9705118

(56) Entgegenhaltungen:
- EP-A- 0 255 047
- WO-A-93/06090
- US-A- 4 979 982

## Beschreibung

Die Erfindung betrifft neue substituierte Phenyluracile, Verfahren zu ihrer Herstellung, neue Zwischenprodukte sowie ihre Verwendung als Herbizide.

Es ist bekannt, daß bestimmte substituierte Uracile herbizide Eigenschaften aufweisen (vgl. WO 93/06090, EP 408382/US 5084084/US 5127935/US5154755, EP 563384, EP 648749, WO 91/00278, US 4979982, US 5169430, DE 4329537). Diese Verbindungen haben jedoch bisher keine nennenswerte Bedeutung erlangt.

Es wurden nun die neuen substituierten Phenyluracile der allgemeinen Formel (I) gefunden 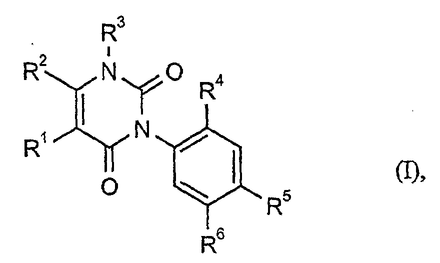 in welcher
- R¹: für Wasserstoff, Fluor, Chlor, Brom oder gegebenenfalls durch Fluor und/oder Chlor substituiertes C₁-C₄-Alkyl steht,
- R²: für gegebenenfalls durch Fluor und/oder Chlor substituiertes C₁-C₄-Alkyl steht,
- R³: für Wasserstoff, Amino, für gegebenenfalls durch Cyano, Fluor, Chlor oder C₁-C₄-Alkoxy substituiertes C₁-C₆-Alkyl oder für gegebenenfalls durch Fluor und/oder Chlor substituiertes C₂-C₆-Alkenyl oder C₂-C₆-Alkinyl steht,
- R⁴: für Wasserstoff, Cyano, Fluor oder Chlor steht,
- R⁵: für Cyano oder Thiocarbamoyl steht, und
- R⁶: für eine der nachstehenden Gruppierungen steht

-C(R⁷,R⁸)-C(R⁷,R⁸)-R⁹ oder -C(R⁷)=C(R⁸)-R⁹

worin
R⁷ und R⁸ gleich oder verschieden sind und unabhängig voneinander für Wasserstoff, Hydroxy, Mercapto, Fluor, Chlor, Brom oder jeweils gegebenenfalls durch Cyano, Fluor, Chlor oder C₁-C₄-Alkoxy substituiertes C₁-C₄-Alkyl, C₁-C₄-Alkoxy oder C₁-C₄-Alkylthio stehen, und
R⁹ für Cyano, Formyl, C₁-C₄-Alkyl-carbonyl, die Gruppierung -CO-OR¹⁰ oder die Gruppierung -CO-N(R¹¹,R¹²) steht, wobei
R¹⁰ für Wasserstoff oder für gegebenenfalls durch Cyano, Fluor, Chlor oder C₁-C₄-Alkoxy substituiertes C₁-C₁₀-Alkyl steht,
R¹⁰ weiterhin für jeweils gegebenenfalls durch Fluor, Chlor oder Brom substituiertes C₃-C₁₀-Alkenyl oder C₃-C₁₀-Alkinyl steht,
R¹⁰ weiterhin für jeweils gegebenenfalls durch Cyano, Fluor, Chlor, Brom, C₁-C₄-Alkyl oder C₁-C₄-Alkoxy-carbonyl substituiertes C₃-C₆-Cycloalkyl oder C₃-C₆-Cycloalkyl-C₁-C₄-alkyl steht,
R¹⁰ weiterhin für jeweils gegebenenfalls durch Cyano, Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder C₁-C₄-Alkoxycarbonyl substituiertes Phenyl, Phenyl-C₁-C₄-alkyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht,
R¹¹ für Wasserstoff oder für jeweils gegebenenfalls durch Cyano, Fluor, Chlor oder C₁-C₄-Alkoxy substituiertes C₁-C₆-Alkyl oder C₁-C₆-Alkoxy steht,
R¹¹ weiterhin für jeweils gegebenenfalls durch Fluor, Chlor oder Brom substituiertes C₃-C₆-Alkenyl oder C₃-C₆-Alkinyl steht, und
R¹² für Wasserstoff oder für gegebenenfalls durch Cyano, Fluor, Chlor oder C₁-C₄-Alkoxy substituiertes C₁-C₁₀-Alkyl steht,
R¹² weiterhin für jeweils gegebenenfalls durch Fluor, Chlor oder Brom substituiertes C₃-C₁₀-Alkenyl oder C₃-C₁₀-Alkinyl steht,
R¹² weiterhin für jeweils gegebenenfalls durch Cyano, Fluor, Chlor, Brom, C₁-C₄-Alkyl oder C₁-C₄-Alkoxy-carbonyl substituiertes C₃-C₆-Cycloalkyl oder C₃-C₆-Cycloalkyl-C₁-C₄-alkyl steht,
R¹² weiterhin für jeweils gegebenenfalls durch Cyano, Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder C₁-C₄-Alkoxycarbonyl substituiertes Phenyl, Phenyl-C₁-C₄-alkyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxadiazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht oder zusammen mit R¹¹ für C₂-C₆-Alkandiyl steht.

Man erhält die neuen substituierten Phenyluracile der allgemeinen Formel (I), wenn man
(a) Aminophenyluracile der allgemeinen Formel (II) 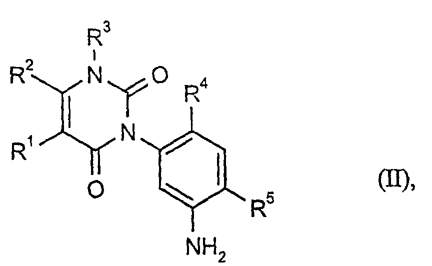 in welcher
   R¹, R², R³, R⁴ und R⁵ die oben angegebenen Bedeutungen haben,
   - oder Säureaddukte von Verbindungen der Formel (II) -
   mit einem Alkalimetall- oder Alkyl-nitrit und mit einem Hydrogenhalogenid (HX¹) oder einem Metallhalogenid (MX¹) gegebenenfalls in Gegenwart eines Verdünnungsmittels umsetzt und die dabei gebildeten Diazoniumsalze der allgemeinen Formel (III) 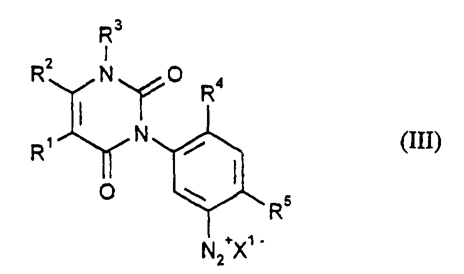 in welcher
   R¹, R², R³, R⁴ und R⁵ die oben angegebenen Bedeutungen haben und
   X¹ für Halogen steht,
   mit Acrylsäurederivaten der allgemeinen Formel (IV)

   C(R⁷,R⁸)=C(R⁷)-R⁹ (IV)

   in welcher
   R⁷, R⁸ und R⁹ die oben angegebenen Bedeutungen haben,
   in Gegenwart von Hydrogenhalogeniden (HX¹), gegebenenfalls in Gegenwart von Katalysatoren, gegebenenfalls in Gegenwart von Wasser und gegebenenfalls in Gegenwart des anfangs verwendeten organischen Losungsmittels umsetzt,
   oder wenn man
(b) substituierte Phenyluracile der allgemeinen Formel (Ia) 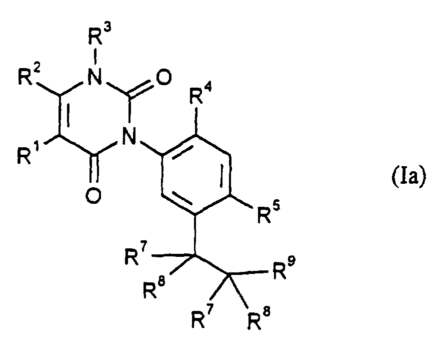 in welcher
   R¹, R², R³, R⁴, R⁵, R⁷, R⁸ und R⁹ die oben angegebenen Bedeutungen haben,
   - wobei jedoch mindestens einer der Reste R⁷ / R⁸ für Wasserstoff steht und mindestens ein weiterer der Reste R⁷ / R⁸ in hierzu vicinaler Position für Halogen steht -
   mit einem Säureakzeptor gegebenenfalls in Gegenwart eines Verdünnungsmittels umsetzt.

Die Verbindungen der allgemeinen Formel (I) können auch nach weiteren üblichen Methoden in andere Verbindungen der allgemeinen Formel (I) gemäß obiger Definition umgewandelt werden, beispielsweise durch Aminierung bzw. Alkylierung (z.B. R³: H → NH₂, H → CH₃), Umsetzung mit Hydrogensulfid (z.B. R⁵ CN → CSNH₂), nucleophile Substitution (z.B. R⁸: Cl → SCH₃), gegebenenfalls Addition von Wasserstoff, Halogen oder Hydrogenhalogenid an eine C-C-Doppelbindung (vgl. Definition von R⁶), Hydrolyse (z.B. R⁹: CN → COOH).
Soweit die erfindungsgemäßen Verbindungen der Formel (I) olefinische Doppelbindungen enthalten (vgl. Definition von R⁶), betrifft die Erfindung sowohl die nach üblichen Methoden trennbaren einzelnen E- und Z- bzw. cis- und trans-Isomeren als auch beliebige Gemische dieser Isomeren.

Die neuen substituierten Phenyluracile der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- R¹: für Wasserstoff, Fluor, Chlor, Brom oder gegebenenfalls durch Fluor und/oder Chlor substituiertes Methyl oder Ethyl steht,
- R²: für gegebenenfalls durch Fluor und/oder Chlor substituiertes Methyl oder Ethyl steht,
- R³: für Wasserstoff, Amino, für gegebenenfalls durch Cyano, Fluor, Chlor, Methoxy oder Ethoxy substituiertes Methyl, Ethyl, n- oder i-Propyl, n-, i-, s- oder t-Butyl, oder für gegebenenfalls durch Fluor und/oder Chlor substituiertes Propenyl, Butenyl, Propinyl oder Butinyl steht,
- R⁴: für Wasserstoff, Fluor oder Chlor steht,
- R⁵: für Cyano oder Thiocarbamoyl steht, und
- R⁶: für eine der nachstehenden Gruppierungen steht

-C(R⁷,R⁸)-C(R⁷,R⁸)-R⁹ oder -C(R⁷)=C(R⁸)-R⁹

worin
R⁷ und R⁸ gleich oder verschieden sind und unabhängig voneinander für Wasserstoff, Fluor, Chlor, Brom oder jeweils gegebenenfalls durch Cyano, Fluor, Chlor, Methoxy oder Ethoxy substituiertes Methyl, Ethyl, Methoxy, Ethoxy, Methylthio oder Ethylthio stehen, und
R⁹ für Cyano, die Gruppierung -CO-OR¹⁰ oder die Gruppierung -CO-N(R¹¹,R¹²) steht, wobei
R¹⁰ für Wasserstoff oder für jeweils gegebenenfalls durch Cyano, Fluor, Chlor, Methoxy oder Ethoxy substituiertes Methyl, Ethyl, n- oder i-Propyl, n-, i-, s- oder t-Butyl, n-, i-, s- oder t-Pentyl steht,
R¹⁰ weiterhin für jeweils gegebenenfalls durch Fluor, Chlor oder Brom substituiertes Propenyl, Butenyl, Pentenyl, Propinyl, Butinyl oder Pentinyl steht,
R¹⁰ weiterhin für jeweils gegebenenfalls durch Cyano, Fluor, Chlor, Brom, Methyl, Ethyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R¹⁰ weiterhin für jeweils gegebenenfalls durch Cyano, Fluor, Chlor, Brom, Methyl, Ethyl, Trifluormethyl, Methoxy, Ethoxy, Difluormethoxy, Trifluormethoxy, Methylthio, Ethylthio, Difluormethylthio, Trifluormethylthio, Methoxycarbonyl, oder Ethoxycarbonyl substituiertes Phenyl, Phenylmethyl, Phenylethyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxadiazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht,
R¹¹ für Wasserstoff oder für jeweils gegebenenfalls durch Cyano, Fluor, Chlor, Methoxy oder Ethoxy substituiertes Methyl, Ethyl, Methoxy oder Ethoxy steht,
R¹¹ weiterhin für jeweils gegebenenfalls durch Fluor, Chlor oder Brom substituiertes Propenyl oder Propinyl steht, und
R¹² für Wasserstoff oder für gegebenenfalls durch Cyano, Fluor, Chlor, Methoxy oder Ethoxy substituiertes Methyl, Ethyl, n- oder i-Propyl, n-, i-, s- oder t-Butyl, n-, i-, s- oder t-Pentyl steht,
R¹² weiterhin für jeweils gegebenenfalls durch Fluor, Chlor oder Brom substituiertes Propenyl, Butenyl, Pentenyl, Propinyl, Butinyl oder Pentinyl, für jeweils gegebenenfalls durch Cyano, Fluor, Chlor, Brom, Methyl, Ethyl, Methoxycarbonyl oder Ethoxy-carbonyl substituiertes Cyclopropyl, Cyclobutyl, Cyclopentyl, Cyclohexyl, Cyclopropylmethyl, Cyclo-butylmethyl, Cyclopentylmethyl oder Cyclohexylmethyl steht, oder
R¹² weiterhin für jeweils gegebenenfalls durch Cyano, Fluor, Chlor, Brom, Methyl, Ethyl, Trifluormethyl, Methoxy, Ethoxy, Difluormethoxy, Trifluormethoxy, Methylthio, Ethylthio, Difluormethylthio, Trifluormethylthio, Methoxycarbonyl, oder Ethoxycarbonyl substituiertes Phenyl, Phenylmethyl, Phenylethyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxadiazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

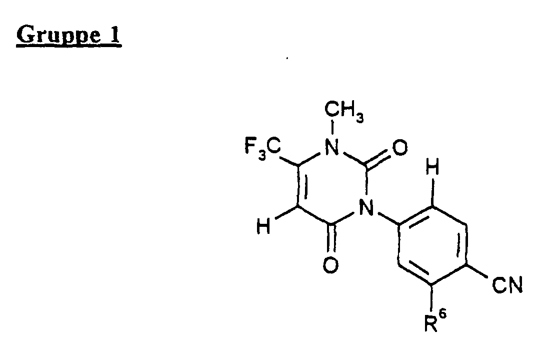

R⁶ hat hierbei beispielhaft die in der nachstehenden Aufzählung angegebenen Bedeutungen:

2-Cyano-ethenyl, 2-Cyano-1-methyl-ethenyl, 2-Cyano-1-chlor-ethenyl, 2-Cyano-2-chlor-ethenyl, 2-Cyano-1-brom-ethenyl, 2-Cyano-2-brom-ethenyl, 2-Cyanopropenyl, 2-Cyano-ethyl, 2-Cyano-2-fluor-ethyl, 2-Cyano-2-chlor-ethyl, 2-Cyano-2-brom-ethyl, 2-Cyano-1,2-dichlor-ethyl, 2-Cyano-1,2-dibrom-ethyl, 2-Cyano-2,2-dichlor-ethyl, 2-Cyano-2-chlor-1-methyl-ethyl, 2-Cyano-2-chlor-1,2-dimethyl-ethyl, 2-Cyano-2-chlor-propyl, 2-Carboxy-ethenyl, 2-Carboxy-1-methyl-ethenyl, 2-Carboxy-1-chlor-ethenyl, 2-Carboxy-2-chlor-ethenyl, 2-Carboxy-1-brom-ethenyl, 2-Carboxy-2-brom-ethenyl, 2-Carboxy-propenyl, 2-Carboxy-ethyl, 2-Carboxy-2-fluorethyl, 2-Carboxy-2-chlor-ethyl, 2-Carboxy-2-brom-ethyl, 2-Carboxy-1,2-dichlorethyl, 2-Carboxy-1,2-dibrom-ethyl, 2-Carboxy-2,2-dichlor-ethyl, 2-Carboxy-2-chlor-1-methyl-ethyl, 2-Carboxy-2-chlor-1,2-dimethyl-ethyl, 2-Carboxy-2-chlorpropyl, 2-Methoxycarbonyl-ethenyl, 2-Methoxycarbonyl-1-methyl-ethenyl, 2-Methoxycarbonyl-1-chlor-ethenyl, 2-Methoxycarbonyl-2-chlor-ethenyl, 2-Methoxycarbonyl-1-brom-ethenyl, 2-Methoxycarbonyl-2-brom-ethenyl, 2-Methoxycarbonylpropenyl, 2-Methoxycarbonyl-ethyl, 2-Methoxycarbonyl-2-fluor-ethyl, 2-Methoxycarbonyl-2-chlor-ethyl, 2-Methoxycarbonyl-2-brom-ethyl, 2-Methoxycarbonyl-1,2-dichlor-ethyl, 2-Methoxycarbonyl-1,2-dibrom-ethyl, 2-Methoxycarbonyl-2,2-dichlor-ethyl, 2-Methoxycarbonyl-2-chlor-1-methyl-ethyl, 2-Methoxycarbonyl-2-chlor-1,2-dimethyl-ethyl, 2-Methoxycarbonyl-2-chlor-propyl, 2-Ethoxycarbonylethenyl, 2-Ethoxycarbonyl-1-methyl-ethenyl, 2-Ethoxycarbonyl-1-chlor-ethenyl, 2-Ethoxycarbonyl-2-chlor-ethenyl, 2-Ethoxycarbonyl-1-brom-ethenyl, 2-Ethoxycarbonyl-2-brom-ethenyl, 2-Ethoxycarbonyl-propenyl, 2-Ethoxycarbonyl-ethyl, 2-Ethoxycarbonyl-2-fluor-ethyl, 2-Ethoxycarbonyl-2-chlor-ethyl, 2-Ethoxycarbonyl-2-brom-ethyl, 2-Ethoxycarbonyl-1,2-dichlor-ethyl, 2-Ethoxycarbonyl-1,2-dibromethyl, 2-Ethoxycarbonyl-2,2-dichlor-ethyl, 2-Ethoxycarbonyl-2-chlor-1-methylethyl, 2-Ethoxycarbonyl-2-chlor-1,2-dimethyl-ethyl, 2-Ethoxycarbonyl-2-chlorpropyl.

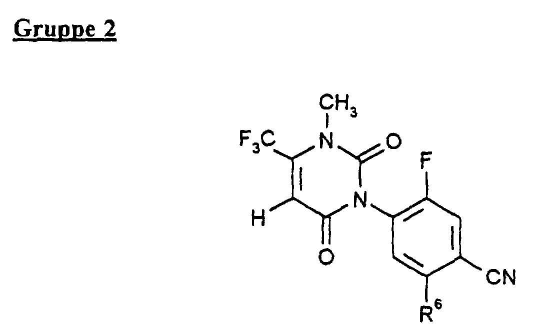

R⁶ hat hierbei beispielhaft die oben in Gruppe 1 aufgeführten Bedeutungen.

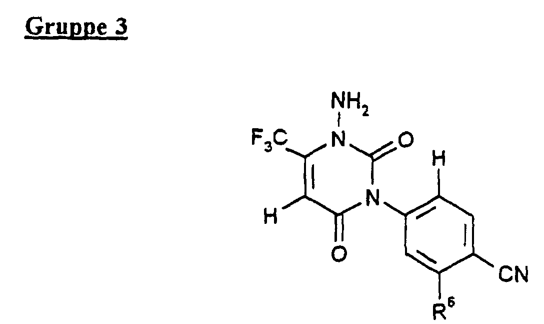

R⁶ hat hierbei beispielhaft die oben in Gruppe 1 aufgeführten Bedeutungen.

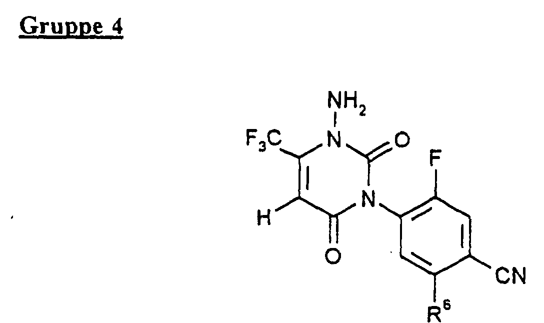

R⁶ hat hierbei beispielhaft die oben in Gruppe 1 aufgeführten Bedeutungen

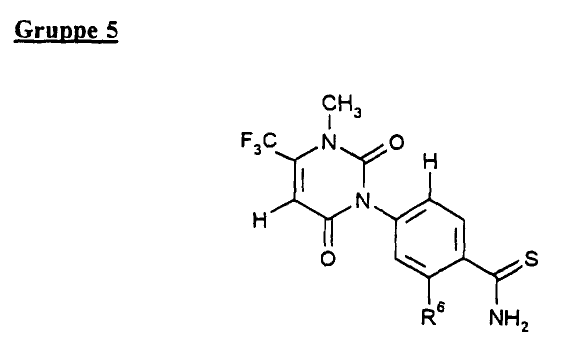

R⁶ hat hierbei beispielhaft die oben in Gruppe 1 aufgeführten Bedeutungen.

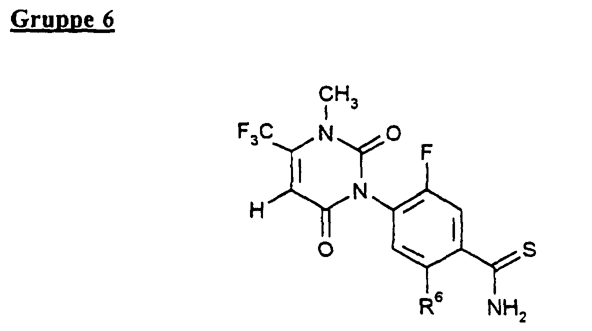

R⁶ hat hierbei beispielhaft die oben in Gruppe 1 aufgeführten Bedeutungen

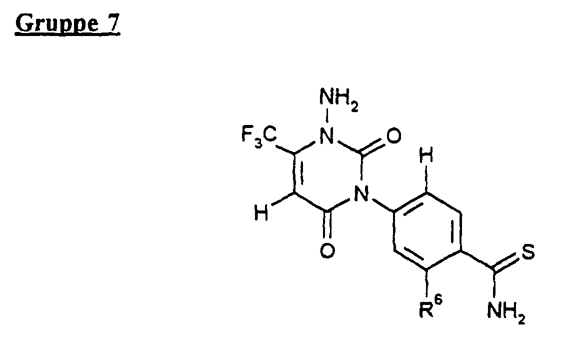

R⁶ hat hierbei beispielhaft die oben in Gruppe 1 aufgeführten Bedeutungen.

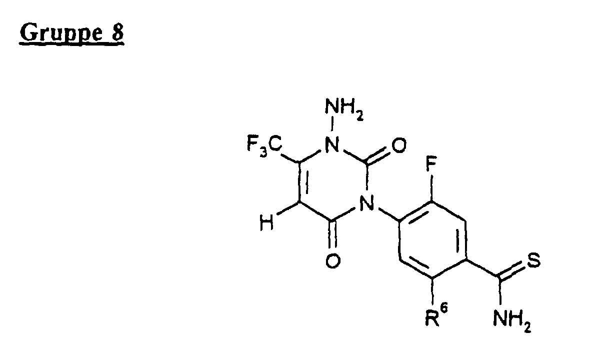

R⁶ hat hierbei beispielhaft die oben in Gruppe 1 aufgeführten Bedeutungen.

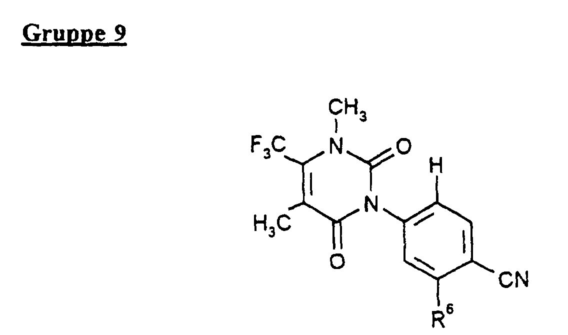

R⁶ hat hierbei beispielhaft die oben in Gruppe 1 aufgeführten Bedeutungen

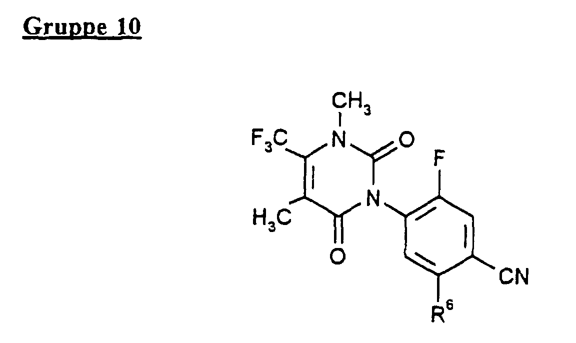

R⁶ hat hierbei beispielhaft die oben in Gruppe 1 aufgeführten Bedeutungen.

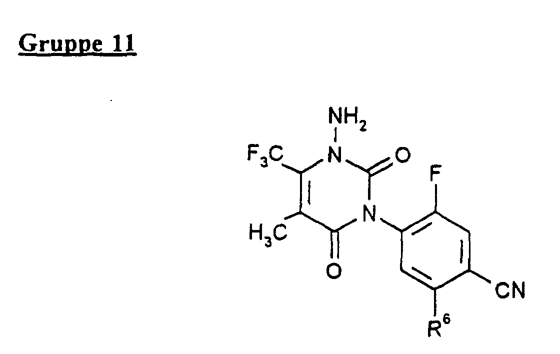

R⁶ hat hierbei beispielhaft die oben in Gruppe 1 aufgeführten Bedeutungen.

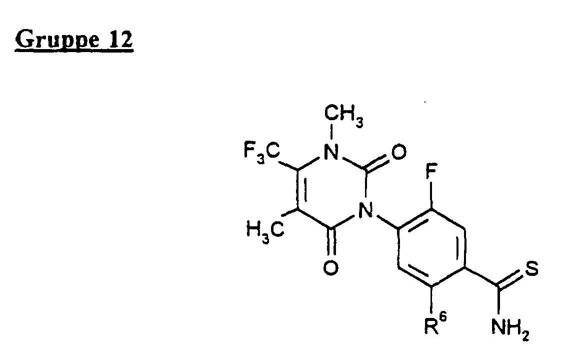

R⁶ hat hierbei beispielhaft die oben in Gruppe 1 aufgeführten Bedeutungen.

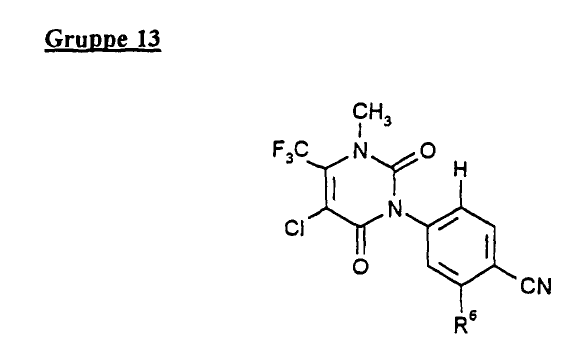

R⁶ hat hierbei beispielhaft die oben in Gruppe 1 aufgeführten Bedeutungen.

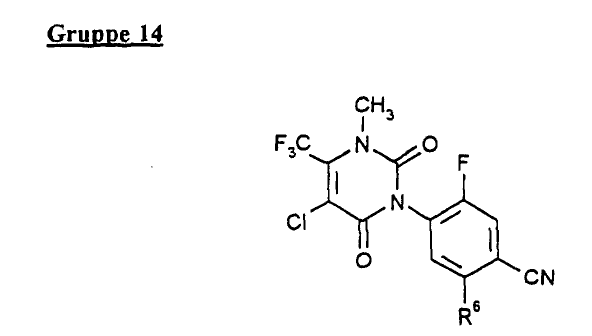

R⁶ hat hierbei beispielhaft die oben in Gruppe 1 aufgeführten Bedeutungen.

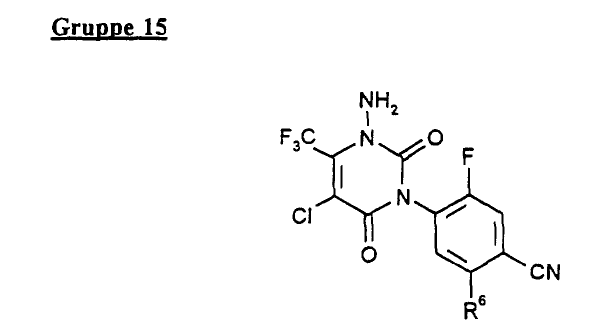

R⁶ hat hierbei beispielhaft die oben in Gruppe 1 aufgeführten Bedeutungen.

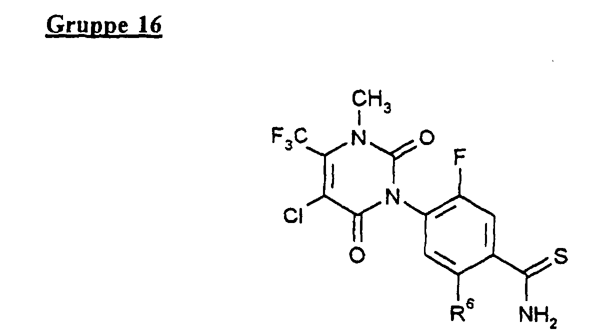

R⁶ hat hierbei beispielhaft die oben in Gruppe 1 aufgeführten Bedeutungen

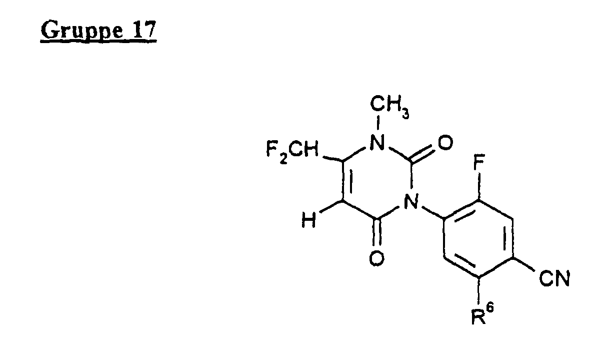

R⁶ hat hierbei beispielhaft die oben in Gruppe 1 aufgeführten Bedeutungen.

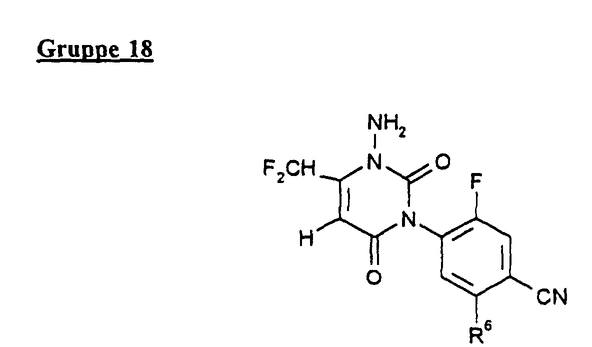

R⁶ hat hierbei beispielhaft die oben in Gruppe 1 aufgeführten Bedeutungen.

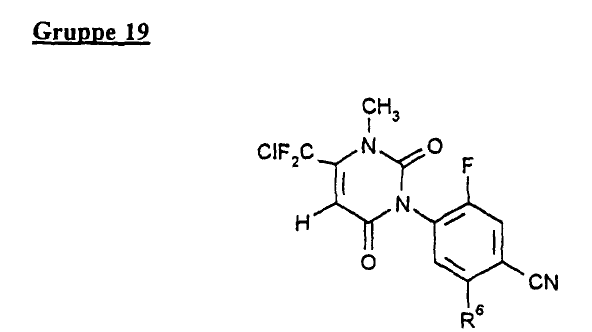

R⁶ hat hierbei beispielhaft die oben in Gruppe 1 aufgeführten Bedeutungen.

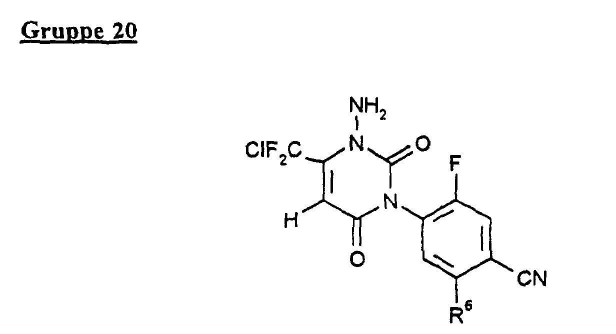

R⁶ hat hierbei beispielhaft die oben in Gruppe 1 aufgeführten Bedeutungen.

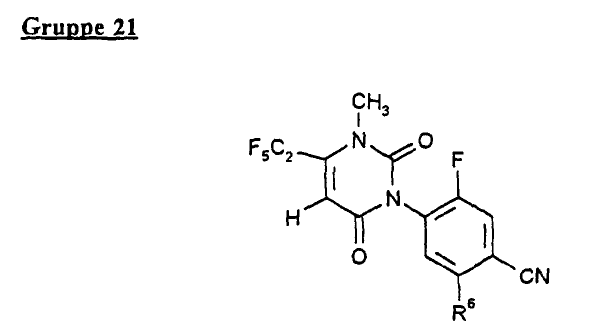

R⁶ hat hierbei beispielhaft die oben in Gruppe 1 aufgeführten Bedeutungen

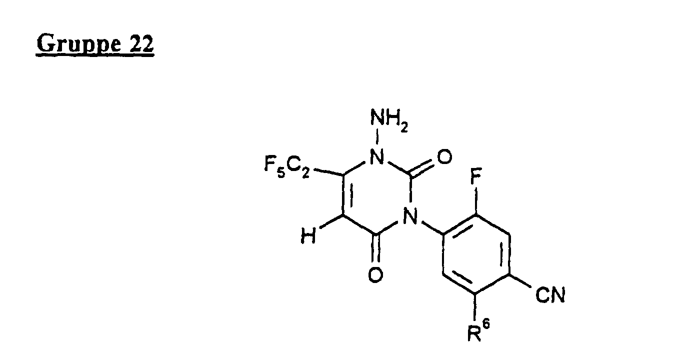

R⁶ hat hierbei beispielhaft die oben in Gruppe 1 aufgefuhrten Bedeutungen.

Verwendet man beispielsweise 1-(5-Amino-4-cyano-2-fluor-phenyl)-3,6-dihydro-2,6-dioxo-3-methyl-4-trifluormethyl-1(2H)-pyrimidin, Natriumnitrit und Salzsäure sowie anschließend Acrylsäure-methylester als Ausgangsstoffe, so kann der Reaktionsablauf beim erfindungsgemäßen Verfahren (a) durch das folgende Formelschema skizziert werden:

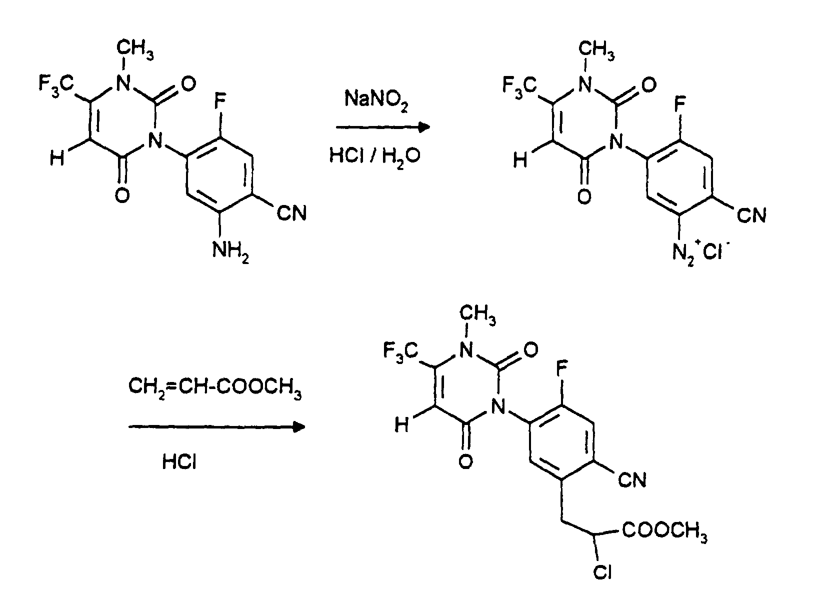

Verwendet man beispielsweise 1-[4-Cyano-2-fluor-5-(2-chlor-2-methoxycarbonylethyl)-phenyl]-3-6-dihydro-2,6-dioxo-3,5-dimethyl-4-trifluormethyl-1(2H)-pyrimidin als Ausgangsstoff und Triethylamin als Säureakzeptor, so kann der Reaktionsablauf beim erfindungsgemäßen Verfahren (b) durch das folgende Formelschema skizziert werden:

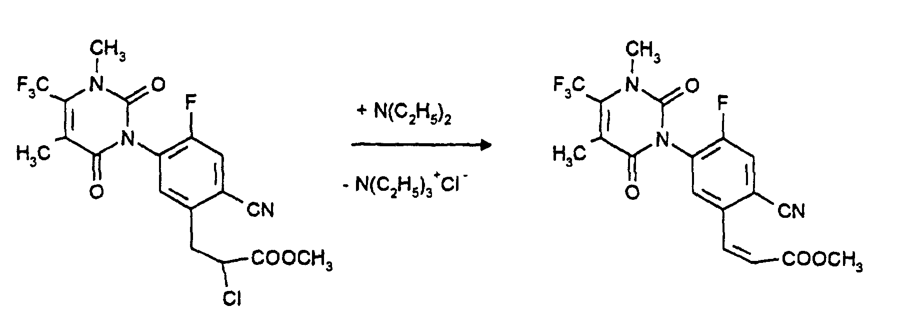

Die beim erfindungsgemäßen Verfahren (a) zur Herstellung der Verbindungen der Formel (I) als Ausgangsstoffe zu verwendenden Aminophenyluracile sind durch die Formel (II) allgemein definiert. In der Formel (II) haben R¹, R², R³, R⁴ und R⁵ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R², R³, R⁴ und R⁵ angegeben wurden.

Die Ausgangsstoffe der Formel (II) sind bekannt und/oder können nach an sich bekannten Verfahren hergestellt werden (vgl. EP 648749, Herstellungsbeispiele).

Die beim erfindungsgemäßen Verfahren (a) zur Herstellung der Verbindungen der Formel (I) weiter als Ausgangsstoffe zu verwendenden Acrylsäurederivate sind durch die Formel (IV) allgemein definiert. In der Formel (IV) haben R⁷, R⁸ und R⁹ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R⁷, R⁸ und R⁹ angegeben wurden.

Die Ausgangsstoffe der Formel (IV) sind bekannte organische Synthesechemikalien.

Die beim erfindungsgemaßen Verfahren (a) zur Herstellung der Verbindungen der Formel (I) intermediär gebildeten Diazoniumsalze sind durch die Formel (III) allgemein definiert. In der Formel (III) haben R¹, R², R³, R⁴ und R⁵ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R², R³, R⁴ und R⁵ angegeben wurden; X¹ steht vorzugsweise für Fluor, Chlor, Brom oder Iod, insbesondere für Chlor oder Brom.

Die beim erfindungsgemäßen Verfahren (b) zur Herstellung der Verbindungen der Formel (I) als Ausgangsstoffe zu verwendenden substituierten Phenyluracile sind durch die Formel (Ia) allgemein definiert. In der Formel (Ia) haben R¹, R², R³, R⁴, R⁵, R⁷, R⁸ und R⁹ vorzugsweise bzw. insbesondere diejenigen Bedeutungen, die bereits oben bei der Beschreibung der erfindungsgemäß herzustellenden Verbindungen der Formel (I) vorzugsweise bzw. als insbesondere bevorzugt für R¹, R², R³, R⁴, R⁵, R⁷, R⁸ und R⁹ angegeben wurden.

Die Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (a) wird unter Einsatz eines Alkalimetall- oder Alkylnitrits durchgeführt. Vorzugsweise kommen hierbei Lithium-, Natrium-, Kalium-, Rubidium- und Cäsium-nitrit, Methyl-, Ethyl-, n- oder i-Propyl-, n-, i-, s- oder t-Butyl-, n-, i-, s- oder t-Pentyl-nitrit, insbesondere Natrium-, Kalium-, Methyl-, n-, i-, s- oder t-Butyl-, n-, i-, s- oder t-Pentyl-nitrit, in Betracht.

Das erfindungsgemäße Verfahren wird unter Einsatz eines Hydrogenhalogenids (HX¹) oder eines Metallhalogenids (MX¹) durchgeführt. Vorzugsweise kommen hierbei Hydrogenfluorid, Hydrogenchlorid, Hydrogenbromid und Hydrogeniodid, Kupfer(I)-chlorid, Kupfer(II)-chlorid und Kupfer(I)-bromid, insbesondere Hydrogenchlorid und Hydrogenbromid, Kupfer(I)-chlorid, Kupfer(II)-chlorid und Kupfer(I)-bromid in Betracht.

Das erfindungsgemäße Verfahren (a) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -i-propylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren (einphasige oder mehrphasige) Gemische mit Wasser oder reines Wasser.

Die Reaktionstemperaturen können bei der Durchführung des erfindungsgemäßen Verfahrens (a) in einem größeren Bereich variiert werden Im allgemeinen arbeitet man bei Temperaturen zwischen -20°C und +100°C, vorzugsweise zwischen -10°C und +80°C, insbesondere zwischen 0°C und 60°C.

Das erfindungsgemäße Verfahren (a) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens (a) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Das erfindungsgemäße Verfahren (b) zur Herstellung der Verbindungen der Formel (I) wird vorzugsweise in Gegenwart eines geeigneten Säureakzeptors durchgeführt. Als Saureakzeptoren kommen im allgemeinen die üblichen anorganischen oder organischen Basen in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8 Diazabicyclo[5,4,0]-undec-7-en (DBU).

Das erfindungsgemäße Verfahren (b) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester,-ethylester, -n- oder -i-propylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder-monoethylether, Diethylenglycol-monomethylether oder -monoethylether; deren Gemische mit Wasser oder reines Wasser.

Die Reaktionstemperaturen können bei der Durchführung des erfindungsgemäßen Verfahrens (b) in einem größeren Bereich variiert werden. Im allgemeinen arbeitet man bei Temperaturen zwischen -10°C und 120°C, vorzugsweise zwischen 0°C und 90°C, insbesondere zwischen 10°C und 60°C.

Das erfindungsgemäße Verfahren (b) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens (b) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgefuhrt und das Reaktionsgemisch wird im allgemeinen mehrere Stunden bei der erforderlichen Temperatur gerührt Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen. Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.
   Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Tragerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuronmethyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

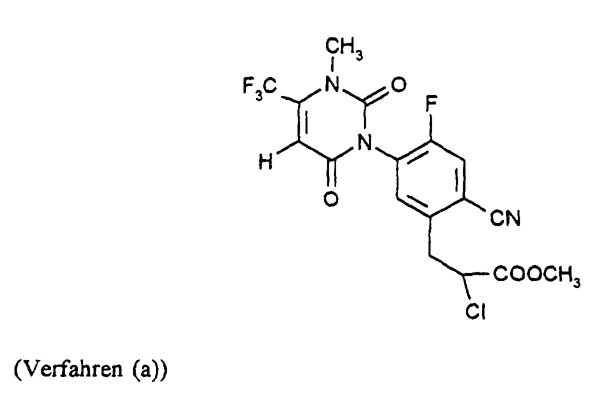

Eine Mischung aus 1,8 g (21 mMol) Acrylsäure-methylester, 1,6 g (15,5 mMol) t-Butylnitrit, 1,6 g (12 mMol) Kupfer(II)-chlorid und 50 ml Acetonitril wird auf ca. 0°C abgekühlt und eine Lösung von 3,3 g (10 mMol) 1-(5-Amino-4-cyano-2-fluorphenyl)-3,6-dihydro-2,6-dioxo-3-methyl-4-trifluormethyl-1(2H)-pyrimidin in 20 ml Acetonitril wird tropfenweise bei dieser Temperatur dazu gegeben. Man lässt dann die Reaktionsmischung auf Raumtemperatur kommen und rührt sie 18 Stunden bei dieser Temperatur. Anschließend wird nach Zugabe von 20 ml 1N-Salzsäure mit Essigsäure-ethylester extrahiert, die organische Phase mit Natriumsulfat getrocknet und filtriert. Das Filtrat wird eingeengt und der Rückstand säulenchromatographisch aufgearbeitet.

Man erhält 2,8 g (65% der Theorie) 1-[4-Cyano-2-fluor-5-(2-chlor-2-methoxycarbonyl-ethyl)-phenyl]-3,6-dihydro-2,6-dioxo-3-methyl-4-trifluormethyl-1(2H)-pyrimidin vom Schmelzpunkt 46°C.

### Beispiel 2

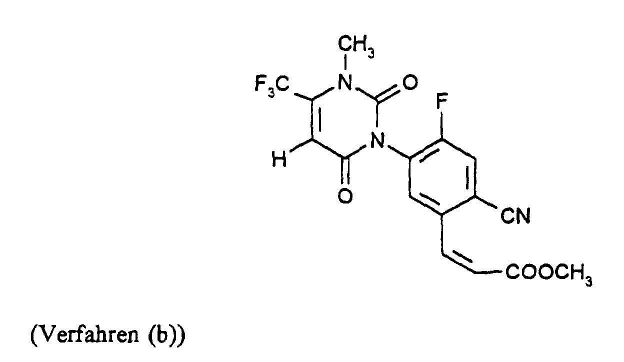

0,22 g Natriumhydrid (60%ig) werden unter Rühren zu einer auf 0°C abgekühlten Mischung aus 2,0 g (4,6 mMol) 1-[4-Cyano-2-fluor-5-(2-chlor-2-methoxycarbonylethyl)-phenyl]-3,6-dihydro-2,6-dioxo-3-methyl-4-trifluormethyl-1(2H)-pyrimidin und 30 ml N,N-Dimethyl-formamid gegeben und die Reaktionsmischung wird zunächst 15 Minuten bei 0°C, dann ca. 60 Minuten bei 20°C und schließlich 6 Stunden bei 60°C gerührt. Dann wird im Wasserstrahlvakuum eingeengt, der Rückstand mit Diisopropylether verrührt und das kristallin angefallene Produkt durch Absaugen isoliert.

Man erhält 1,1 g (60% der Theorie) 1-[4-Cyano-2-fluor-5-(2-methoxycarbonylethenyl)-phenyl]-3,6-dihydro-2,6-dioxo-3-methyl-4-trifluormethyl-1(2H)-pyrimidin vom Schmelzpunkt 154°C.

Analog Beispiel 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

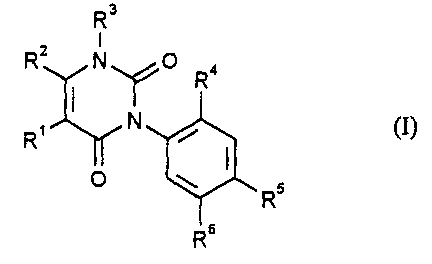

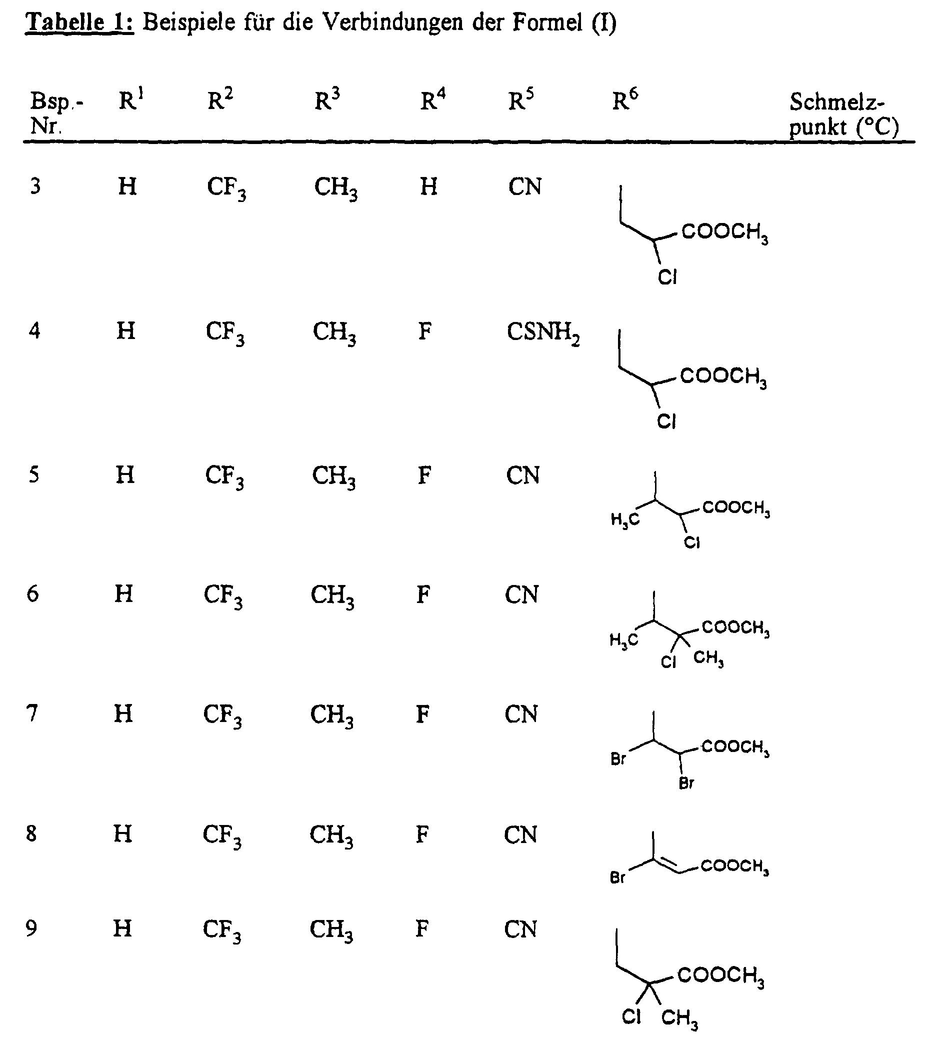

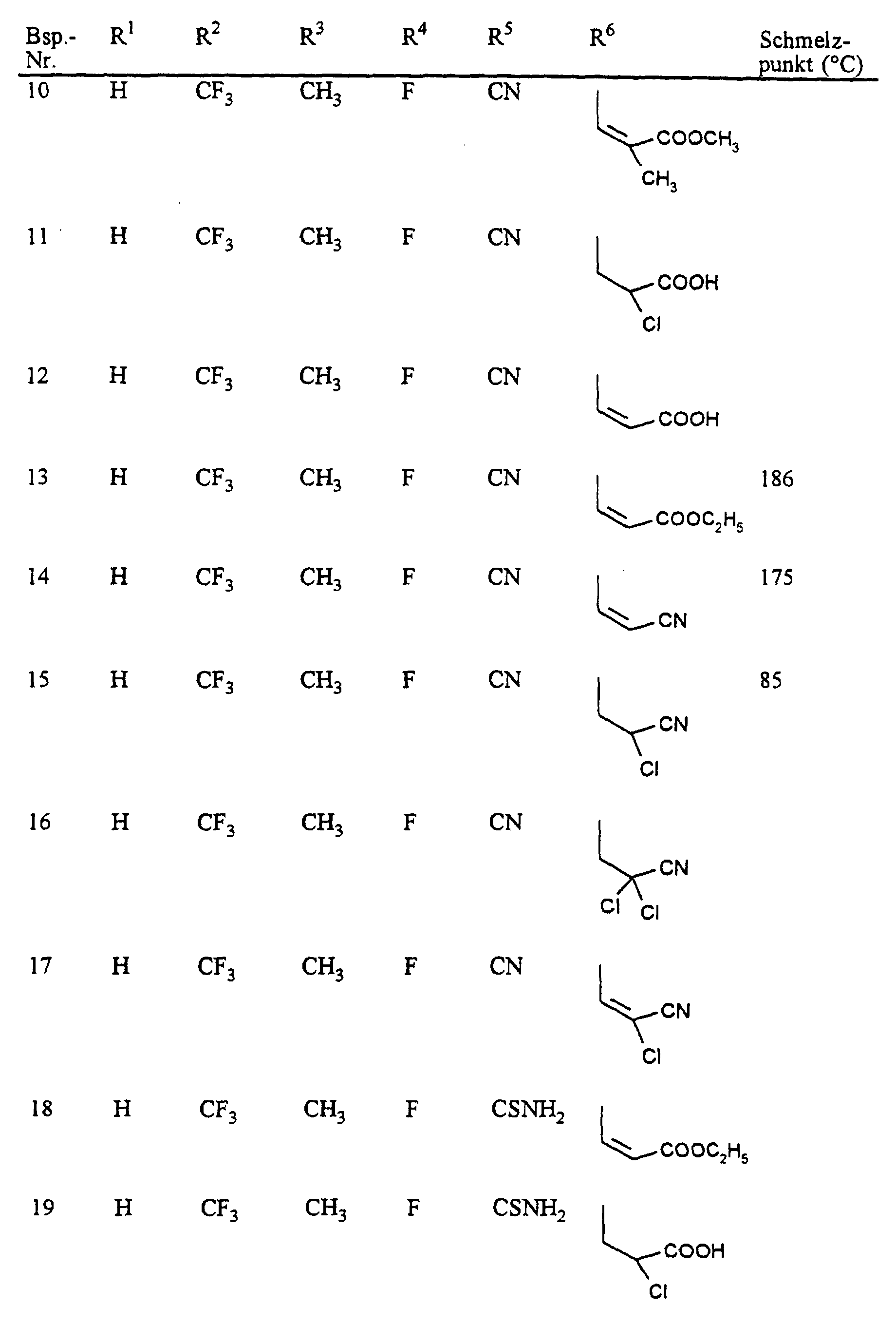

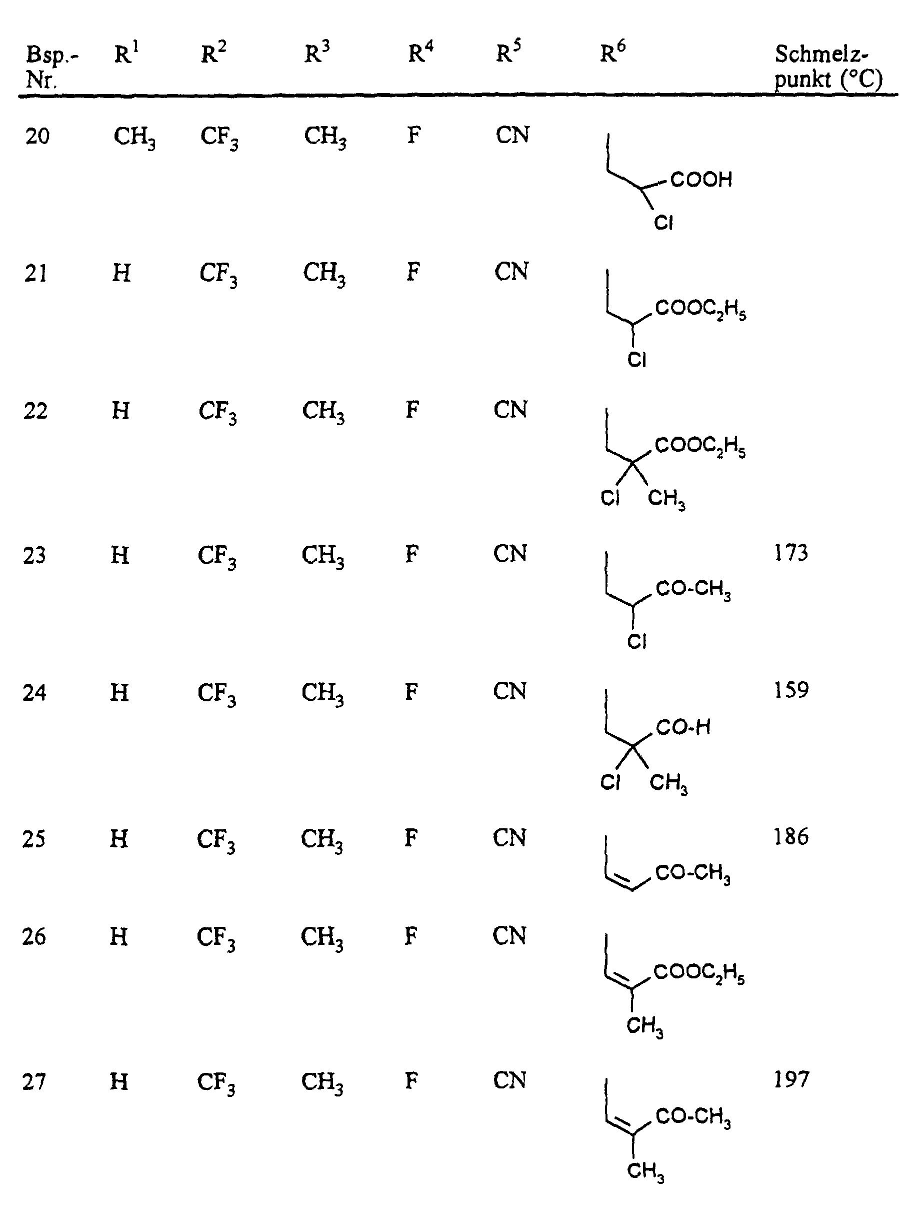

### Ausgangsstoffe der Formel (II):

### Beispiel (II-1)

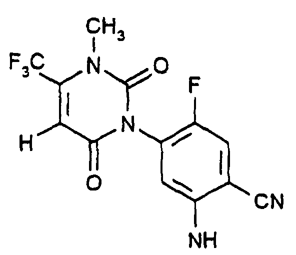

0,17 g (1,2 mMol) Pivalinsäurechlorid werden unter Rühren zu einer Mischung aus 0,50 g (1,2 mMol) 1-(4-Cyano-2-fluor-5-trifluoracetylamino-phenyl)-3,6-dihydro-2,6-dioxo-3-methyl-4-trifluormethyl-1(2H)-pyrimidin, 1 ml Triethylamin und 50 ml Acetonitril gegeben und die Reaktionsmischung wird 18 Stunden bei 20°C und weitere 15 Stunden bei 60°C gerührt. Dann wird im Wasserstrahlvakuum eingeengt, der Rückstand mit 1N-Salzsaure/Essigsäureethylester geschüttelt, die organische Phase abgetrennt, mit Natriumsulfat getrocknet und filtriert. Das Filtrat wird im Wasserstrahlvakuum eingeengt und der Rückstand säulenchromatographisch (Kieselgel, Chloroform/Essigsäureethylester, Vol.: 1:1) aufgearbeitet.

Man erhält neben nicht umgesetztem 1-(4-Cyano-2-fluor-5-trifluoracetylamino-phenyl)-3,6-dihydro-2,6-dioxo-3-methyl-4-trifluormethyl-1(2H)-pyrimidin (erste Fraktion: 0,30 g) 0,2 g (50% der Theorie) 1-(4-Cyano-2-fluor-5-amino-phenyl)-3,6-dihydro-2,6-dioxo-3-methyl-4-trifluormethyl-1(2H)-pyrimidin als zweite Fraktion.
Schmelzpunkt: 195°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Dieser wird nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen die Verbindungen der Formel (I) starke Wirkung gegen Unkräuter.

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß inca. 1000 l/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen die Verbindungen gemäß der Herstellungsbeispiele 1 und 2 bei Aufwandmengen von 125 g/ha starke Wirkung gegen Unkräuter wie Abutilon (100 %), Amaranthus (100 %), Galium (100 %), Xanthium (100 %), Setaria (100 %) sowie Avena fatua (95 %).

## Patentansprüche

1. Substituierte Phenyluracile der allgemeinen Formel (I) 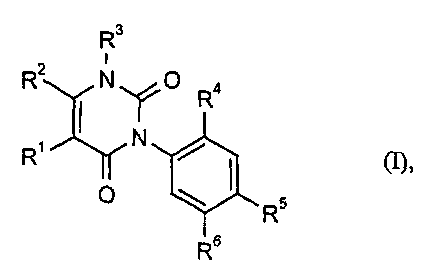 in welcher
R¹ für Wasserstoff, Fluor, Chlor, Brom oder gegebenenfalls durch Fluor und/oder Chlor substituiertes C₁-C₄-Alkyl steht,
R² für gegebenenfalls durch Fluor und/oder Chlor substituiertes C₁-C₄-Alkyl steht,
R³ für Wasserstoff, Amino, für gegebenenfalls durch Cyano, Fluor, Chlor oder C₁-C₄-Alkoxy substituiertes C₁-C₆-Alkyl oder für gegebenenfalls durch Fluor und/oder Chlor substituiertes C₂-C₆-Alkenyl oder C₂-C₆-Alkinyl steht,
R⁴ für Wasserstoff, Cyano, Fluor oder Chlor steht,
R⁵ für Cyano oder Thiocarbamoyl steht, und
R⁶ für eine der nachstehenden Gruppierungen steht
-C(R⁷,R⁸)-C(R⁷,R⁸)-R⁹ oder -C(R⁷)=C(R⁸)-R⁹
worin
R⁷ und R⁸ gleich oder verschieden sind und unabhängig voneinander für Wasserstoff, Hydroxy, Mercapto, Fluor, Chlor, Brom oder jeweils gegebenenfalls durch Cyano, Fluor, Chlor oder C₁-C₄-Alkoxy substituiertes C₁-C₄-Alkyl, C₁-C₄-Alkoxy oder C₁-C₄-Alkylthio stehen, und
R⁹ für Cyano, Formyl, C₁-C₄-Alkyl-carbonyl, die Gruppierung-CO-OR¹⁰ oder die Gruppierung -CO-N(R¹¹,R¹²) steht, wobei
R¹⁰ für Wasserstoff oder für gegebenenfalls durch Cyano, Fluor, Chlor oder C₁-C₄-Alkoxy substituiertes C₁-C₁₀-Alkyl steht,
R¹⁰ weiterhin für jeweils gegebenenfalls durch Fluor, Chlor oder Brom substituiertes C₃-C₁₀-Alkenyl oder C₃-C₁₀-Alkinyl steht,
R¹⁰ weiterhin für jeweils gegebenenfalls durch Cyano, Fluor, Chlor, Brom, C₁-C₄-Alkyl oder C₁-C₄-Alkoxy-carbonyl substituiertes C₃-C₆-Cycloalkyl oder C₃-C₆-Cycloalkyl-C₁-C₄-alkyl steht,
R¹⁰ weiterhin für jeweils gegebenenfalls durch Cyano, Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder C₁-C₄-Alkoxy-carbonyl substituiertes Phenyl, Phenyl-C₁-C₄-alkyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht,
R¹¹ für Wasserstoff oder für jeweils gegebenenfalls durch Cyano, Fluor, Chlor oder C₁-C₄-Alkoxy substituiertes C₁-C₆-Alkyl oder C₁-C₆-Alkoxy steht,
R¹¹ weiterhin für jeweils gegebenenfalls durch Fluor, Chlor oder Brom substituiertes C₃-C₆-Alkenyl oder C₃-C₆-Alkinyl steht, und
R¹² für Wasserstoff oder für gegebenenfalls durch Cyano, Fluor, Chlor oder C₁-C₄-Alkoxy substituiertes C₁-C₁₀-Alkyl steht,
R¹² weiterhin für jeweils gegebenenfalls durch Fluor, Chlor oder Brom substituiertes C₃-C₁₀-Alkenyl oder C₃-C₁₀-Alkinyl steht,
R¹² weiterhin für jeweils gegebenenfalls durch Cyano, Fluor, Chlor, Brom, C₁-C₄-Alkyl oder C₁-C₄-Alkoxy-carbonyl substituiertes C₃-C₆-Cycloalkyl oder C₃-C₆-Cycloalkyl-C₁-C₄-alkyl steht,
R¹² weiterhin für jeweils gegebenenfalls durch Cyano, Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder C₁-C₄-Alkoxy-carbonyl substituiertes Phenyl, Phenyl-C₁-C₄-alkyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht oder zusammen mit R¹¹ für C₂-C₆-Alkandiyl steht.

2. Substituierte Phenyluracile der allgemeinen Formel (I) gemäß Anspruch 1,
**dadurch gekennzeichnet, daß**
R¹ für Wasserstoff, Fluor, Chlor, Brom oder gegebenenfalls durch Fluor und/oder Chlor substituiertes Methyl oder Ethyl steht,
R² für gegebenenfalls durch Fluor und/oder Chlor substituiertes Methyl oder Ethyl steht,
R³ für Wasserstoff, Amino, für gegebenenfalls durch Cyano, Fluor, Chlor, Methoxy oder Ethoxy substituiertes Methyl, Ethyl, n- oder i-Propyl, n-, i-, s- oder t-Butyl, oder für gegebenenfalls durch Fluor und/oder Chlor substituiertes Propenyl, Butenyl, Propinyl oder Butinyl steht,
R⁴ für Wasserstoff, Fluor oder Chlor steht,
R⁵ für Cyano oder Thiocarbamoyl steht, und
R⁶ für eine der nachstehenden Gruppierungen steht
-C(R⁷,R⁸)-C(R⁷,R⁸)-R⁹ oder -C(R⁷)=C(R⁸)-R⁹
worin
R⁷ und R⁸ gleich oder verschieden sind und unabhängig voneinander für Wasserstoff, Fluor, Chlor, Brom oder jeweils gegebenenfalls durch Cyano, Fluor, Chlor, Methoxy oder Ethoxy substituiertes Methyl, Ethyl, Methoxy, Ethoxy, Methylthio oder Ethylthio stehen, und
R⁹ für Cyano, die Gruppierung -CO-OR¹⁰ oder die Gruppierung -CO-N(R¹¹,R¹²) steht, wobei
R¹⁰ für Wasserstoff oder für gegebenenfalls durch Cyano, Fluor, Chlor, Methoxy oder Ethoxy substituiertes Methyl, Ethyl, n- oder i-Propyl, n-, i-, s- oder t-Butyl, n-, i-, s- oder t-Pentyl steht,
R¹⁰ weiterhin für jeweils gegebenenfalls durch Fluor, Chlor oder Brom substituiertes Propenyl, Butenyl, Pentenyl, Propinyl, Butinyl oder Pentinyl steht,
R¹⁰ weiterhin für jeweils gegebenenfalls durch Cyano, Fluor, Chlor, Brom, Methyl, Ethyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R¹⁰ weiterhin für jeweils gegebenenfalls durch Cyano, Fluor, Chlor, Brom, Methyl, Ethyl, Trifluormethyl, Methoxy, Ethoxy, Difluormethoxy, Trifluormethoxy, Methylthio, Ethylthio, Difluormethylthio, Trifluormethylthio, Methoxycarbonyl, oder Ethoxy-carbonyl substituiertes Phenyl, Phenylmethyl, Phenylethyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxazolylmethyl, Thiazolyl, Thiazolylmethyl, Oxadiazolyl, Oxadiazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht,
R¹¹ für Wasserstoff oder für jeweils gegebenenfalls durch Cyano, Fluor, Chlor, Methoxy oder Ethoxy substituiertes Methyl, Ethyl, Methoxy oder Ethoxy steht,
R¹¹ weiterhin für jeweils gegebenenfalls durch Fluor, Chlor oder Brom substituiertes Propenyl oder Propinyl steht, und
R¹² für Wasserstoff oder für gegebenenfalls durch Cyano, Fluor, Chlor, Methoxy oder Ethoxy substituiertes Methyl, Ethyl, n- oder i-Propyl, n-, i-, s- oder t-Butyl, n-, i-, s- oder t-Pentyl steht,
R¹² weiterhin für jeweils gegebenenfalls durch Fluor, Chlor oder Brom substituiertes Propenyl, Butenyl, Pentenyl, Propinyl, Butinyl oder Pentinyl, für jeweils gegebenenfalls durch Cyano, Fluor, Chlor, Brom, Methyl, Ethyl, Methoxycarbonyl oder Ethoxy-carbonyl substituiertes Cyclopropyl, Cyclobutyl, Cyclopentyl, Cyclohexyl, Cyclopropylmethyl, Cyclo-butylmethyl, Cyclopentylmethyl oder Cyclohexylmethyl steht,
R¹² weiterhin für jeweils gegebenenfalls durch Cyano, Fluor, Chlor, Brom, Methyl, Ethyl, Trifluormethyl, Methoxy, Ethoxy, Difluormethoxy, Trifluormethoxy, Methylthio, Ethylthio, Difluormethylthio, Trifluormethylthio, Methoxycarbonyl, oder Ethoxy-carbonyl substituiertes Phenyl, Phenylmethyl, Phenylethyl, Furyl, Furylmethyl, Tetrahydrofuryl, Tetrahydrofurylmethyl, Thienyl, Thienylmethyl, Tetrahydrothienyl, Tetrahydrothienylmethyl, Perhydropyranyl, Perhydropyranylmethyl, Oxazolyl, Oxadiazolylmethyl, Thiazolyl, Thiazolylmethyl, Oxadiazolyl, Oxazolylmethyl, Thiadiazolyl, Thiadiazolylmethyl, Dioxolanyl, Dioxolanylmethyl, Pyridinyl, Pyridinylmethyl, Pyrimidinyl oder Pyrimidinylmethyl steht.

3. Verfahren zur Herstellung substituierter Phenyluracile der allgemeinen Formel (I) 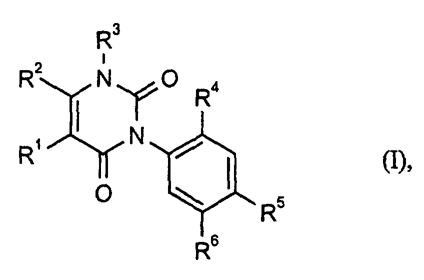 in welcher
R¹, R², R³, R⁴, R⁵ und R⁶ die in Anspruch 1 genannten Bedeutungen haben,
**dadurch gekennzeichnet, daß** man
(a) Aminophenyluracile der allgemeinen Formel (II) 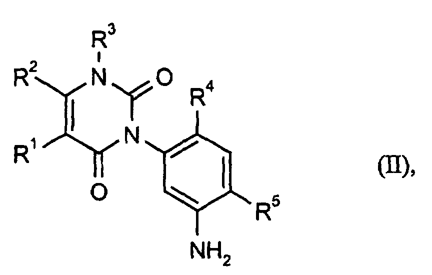 in welcher
R¹, R², R³, R⁴ und R⁵ die oben angegebenen Bedeutungen haben,
- oder Säureaddukte von Verbindungen der Formel (II) -
mit einem Alkalimetall- oder Alkyl-nitrit und mit einem Hydrogenhalogenid (HX¹) oder einem Metallhalogenid (MX¹) gegebenenfalls in Gegenwart eines Verdünnungsmittels umsetzt und die dabei gebildeten Diazoniumsalze der allgemeinen Formel (III) 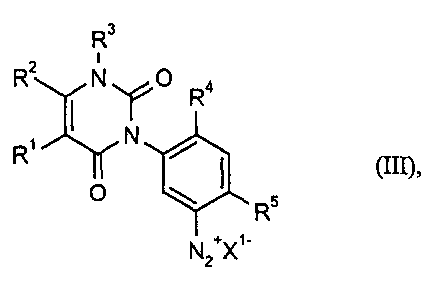 in welcher
R¹, R², R³, R⁴ und R⁵ die oben angegebenen Bedeutungen haben und
X¹ für Halogen steht,
mit Acrylsäurederivaten der allgemeinen Formel (IV)
C(R⁷,R⁸)=C(R⁷)-R⁹ (IV)
in welcher
R⁷, R⁸ und R⁹ die oben angegebenen Bedeutungen haben,
in Gegenwart von Hydrogenhalogeniden (HX¹), gegebenenfalls in Gegenwart von Katalysatoren, gegebenenfalls in Gegenwart von Wasser und gegebenenfalls in Gegenwart des anfangs verwendeten organischen Lösungsmittels umsetzt,
oder daß man
(b) substituierte Phenyluracile der allgemeinen Formel (Ia) 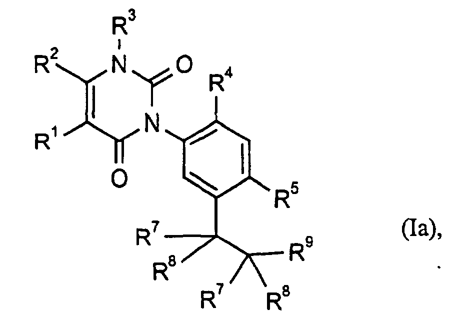 in welcher
R¹, R², R³, R⁴, R⁵, R⁷, R⁸ und R⁹ die oben angegebenen Bedeutungen haben,
- wobei jedoch mindestens einer der Reste R⁷ / R⁸ für Wasserstoff steht und mindestens ein weiterer der Reste R⁷ / R⁸ in hierzu vicinaler Position für Halogen steht -
mit einem Säureakzeptor gegebenenfalls in Gegenwart eines Verdünnungsmittels umsetzt.

4. Substituierte Phenyluracile der allgemeinen Formel (Ia) 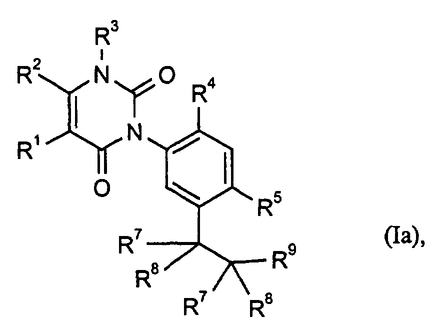 in welcher
R¹ für Wasserstoff, Halogen oder gegebenenfalls substituiertes Alkyl steht,
R² für gegebenenfalls substituiertes Alkyl steht,
R³ für Wasserstoff, Amino oder jeweils gegebenenfalls substituiertes Alkyl, Alkenyl oder Alkinyl steht,
R⁴ für Wasserstoff, Cyano oder Halogen steht,
R⁵ für Cyano oder Thiocarbamoyl steht, und
R⁷ und R⁸ gleich oder verschieden sind und unabhängig voneinander für Wasserstoff, Hydroxy, Mercapto, Halogen oder jeweils gegebenenfalls substituiertes Alkyl, Alkoxy oder Alkylthio stehen, und
R⁹ für Cyano, die Gruppierung -CO-OR¹⁰ oder die Gruppierung -CO-N(R¹¹,R¹²) steht, wobei
R¹⁰ für Wasserstoff oder für einen jeweils gegebenenfalls substituierten Rest der Reihe Alkyl, Alkenyl, Alkinyl, Cycloalkyl, Cycloalkylalkyl, Aryl, Arylalkyl, Heterocyclyl oder Heterocyclylalkyl steht,
R¹¹ für Wasserstoff oder für einen jeweils gegebenenfalls substituierten Rest der Reihe Alkyl, Alkoxy, Alkenyl oder Alkinyl steht, und
R¹² für Wasserstoff oder für einen jeweils gegebenenfalls substituierten Rest der Reihe Alkyl, Alkenyl, Alkinyl, Cycloalkyl, Cycloalkylalkyl, Aryl, Arylalkyl, Heterocyclyl oder Heterocyclylalkyl steht oder zusammen mit R¹ für Alkandiyl steht.

5. Herbizide Mittel, **gekennzeichnet durch** einen Gehalt an mindestens einem substituierten Phenyluracil der allgemeinen Formel (I) gemäß den Ansprüchen 1 bis 4.

6. Verfahren zur Bekämpfung von unerwünschten Pflanzen, **dadurch gekennzeichnet, daß** man substituierte Phenyluracile der allgemeinen Formel (I) gemäß den Ansprüchen 1 bis 4 auf unerwünschte Pflanzen und/oder ihren Lebensraum einwirken läßt.

7. Verwendung von substituierten Phenyluracilen der allgemeinen Formel (I) gemäß der Ansprüche 1 bis 4 zur Bekämpfung von unerwünschten Pflanzen.

8. Verfahren zur Herstellung von herbiziden Mitteln, **dadurch gekennzeichnet, daß** man substituierte Phenyluracile der allgemeinen Formel (I) gemäß der Ansprüche 1 bis 4 mit Streckmitteln und/oder oberflächenaktiven Substanzen vermischt.

## Claims

1. Substituted phenyluracils of the general formula (I) 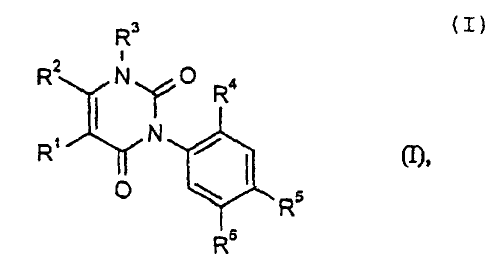 in which
R¹ represents hydrogen, fluorine, chlorine, bromine or optionally fluorine- and/or chlorine-substituted C₁-C₄-alkyl,
R² represents optionally fluorine- and/or chlorine-substituted C₁-C₄-alkyl,
R³ represents hydrogen, amino, represents optionally cyano-, fluorine-, chlorine- or C₁-C₄-alkoxy-substituted C₁-C₆-alkyl or represents optionally fluorine- and/or chlorine-substituted C₂-C₆-alkenyl or C₂-C₆-alkinyl,
R⁴ represents hydrogen, cyano, fluorine or chlorine,
R⁵ represents cyano or thiocarbamoyl, and
R⁶ represents one of the groupings below
-C(R⁷,R⁸)-C(R⁷,R⁸)-R⁹ or -C(R⁷)=C(R⁸)-R⁹
in which
R⁷ and R⁸ are identical or different and each represents independently of the other hydrogen, hydroxyl, mercapto, fluorine, chlorine, bromine or respectively optionally cyano-, fluorine-, chlorine- or C₁-C₄-alkoxy-substituted C₁-C₄-alkyl, C₁-C₄-alkoxy or C₁-C₄- alkylthio, and
R⁹ represents cyano, formyl, C₁-C₄-alkylcarbonyl, the grouping -CO-OR¹⁰ or the grouping
-CO-N(R¹¹,R¹²), where
R¹⁰ represents hydrogen or represents optionally cyano-, fluorine-, chlorine- or C₁-C₄-alkoxy-substituted C₁-C₁₀-alkyl,
R¹⁰ furthermore represents respectively optionally fluorine-, chlorine- or bromine-substituted C₃-C₁₀-alkenyl or C₃-C₁₀-alkinyl,
R¹⁰ furthermore represents respectively optionally cyano-, fluorine-, chlorine-, bromine-, C₁-C₄-alkyl- or C₁-C₄-alkoxy-carbonyl-substituted C₃-C₆-cycloalkyl or C₃-C₆-cycloalkyl-C₁-C₄-alkyl,
R¹⁰ furthermore represents respectively optionally cyano-, fluorine-, chlorine-, bromine-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio- or C₁-C₄-alkoxycarbonyl-substituted phenyl, phenyl-C₁-C₄-alkyl, furyl, furylmethyl, tetrahydrofuryl, tetrahydrofurylmethyl, thienyl, thienylmethyl, tetrahydrothienyl, tetrahydrothienylmethyl, perhydropyranyl, perhydropyranylmethyl, oxazolyl, oxazolylmethyl, thiazolyl, thiazolylmethyl, oxadiazolyl, oxadiazolylmethyl, thiadiazolyl, thiadiazolylmethyl, dioxolanyl, dioxolanylmethyl, pyridinyl, pyridinylmethyl, pyrimidinyl or pyrimidinylmethyl,
R¹¹ represents hydrogen or represents respectively optionally cyano-, fluorine-, chlorine- or C₁-C₄-alkoxy-substituted C₁-C₆-alkyl or C₁-C₆-alkoxy,
R¹¹ furthermore represents respectively optionally fluorine-, chlorine- or bromine-substituted C₃-C₆-alkenyl or C₃-C₆-alkinyl, and
R¹² represents hydrogen or represents optionally cyano-, fluorine-, chlorine- or C₁-C₄-alkoxy-substituted C₁-C₁₀-alkyl,
R¹² furthermore represents respectively optionally fluorine-, chlorine- or bromine-substituted C₃-C₁₀-alkenyl or C₃-C₁₀-alkinyl,
R¹² furthermore represents respectively optionally cyano-, fluorine-, chlorine-, bromine-, C₁-C₄-alkyl- or C₁-C₄-alkoxycarbonyl-substituted C₃-C₆-cycloalkyl or C₃-C₆-cycloalkyl-C₁-C₄-alkyl,
R¹² furthermore represents respectively optionally cyano-, fluorine-, chlorine-, bromine-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio- or C₁-C₄-alkoxycarbonyl-substituted phenyl, phenyl-C₁-C₄-alkyl, furyl, furylmethyl, tetrahydrofuryl, tetrahydrofurylmethyl, thienyl, thienylmethyl, tetrahydrothienyl, tetrahydrothienylmethyl, perhydropyranyl, perhydropyranylmethyl, oxazolyl, oxazolylmethyl, thiazolyl, thiazolylmethyl, oxadiazolyl, oxadiazolylmethyl, thiadiazolyl, thiadiazolylmethyl, dioxolanyl, dioxolanylmethyl, pyridinyl, pyridinylmethyl, pyrimidinyl or pyrimidinylmethyl or together with R¹¹ represents C₂-C₆-alkanediyl.

2. Substituted phenyluracils of the general formula (I) according to Claim 1, **characterized in that**
R¹ represents hydrogen, fluorine, chlorine, bromine or optionally fluorine- and/or chlorine-substituted methyl or ethyl,
R² represents optionally fluorine- and/or chlorine-substituted methyl or ethyl,
R³ represents hydrogen, amino, represents optionally cyano-, fluorine-, chlorine-, methoxy- or ethoxy-substituted methyl, ethyl, n- or i-propyl, n-, i-, s- or t-butyl, or represents optionally fluorine- and/or chlorine-substituted propenyl, butenyl, propinyl or butinyl,
R⁴ represents hydrogen, fluorine or chlorine,
R⁵ represents cyano or thiocarbamoyl, and
R⁶ represents one of the groupings below
-C(R⁷,R⁸)-C(R⁷,R⁸)-R⁹ or -C(R⁷)=C(R⁸)-R⁹
in which
R⁷ and R⁸ are identical or different and each represents independently of the other hydrogen, fluorine, chlorine, bromine or respectively optionally cyano-, fluorine-, chlorine-, methoxy- or ethoxy-substituted methyl, ethyl, methoxy, ethoxy, methylthio or ethylthio, and
R⁹ represents cyano, the grouping -CO-OR¹⁰ or the grouping -CO-N(R¹¹,R¹²), where
R¹⁰ represents hydrogen or represents optionally cyano-, fluorine-, chlorine-, methoxy- or ethoxy-substituted methyl, ethyl, n- or i-propyl, n-, i-, s- or t-butyl, n-, i-, s- or t-pentyl,
R¹⁰ furthermore represents respectively optionally fluorine-, chlorine- or bromine-substituted propenyl, butenyl, pentenyl, propinyl, butinyl or pentinyl,
R¹⁰ furthermore represents respectively optionally cyano-, fluorine-, chlorine-, bromine-, methyl-, ethyl-, methoxycarbonyl- or ethoxycarbonyl-substituted cyclopropyl, cyclobutyl, cyclopentyl, cyclohexyl, cyclopropylmethyl, cyclobutylmethyl, cyclopentylmethyl or cyclohexylmethyl,
R¹⁰ furthermore represents respectively optionally cyano-, fluorine-, chlorine-, bromine-, methyl-, ethyl-, trifluoromethyl-, methoxy-, ethoxy-, difluoromethoxy-, trifluoromethoxy-, methylthio-, ethylthio-, difluoromethylthio-, trifluoromethyl-, methoxycarbonyl- or ethoxycarbonyl-substituted phenyl, phenylmethyl, phenylethyl, furyl, furylmethyl, tetrahydrofuryl, tetrahydrofurylmethyl, thienyl, thienylmethyl, tetrahydrothienyl, tetrahydrothienylmethyl, perhydropyranyl, perhydropyranylmethyl, oxazolyl, oxazolylmethyl, thiazolyl, thiazolylmethyl, oxadiazolyl, oxadiazolylmethyl, thiadiazolyl, thiadiazolylmethyl, dioxolanyl, dioxolanylmethyl, pyridinyl, pyridinylmethyl, pyrimidinyl or pyrimidinylmethyl,
R¹¹ represents hydrogen or represents respectively optionally cyano-, fluorine-, chlorine-, methoxy- or ethoxy-substituted methyl, ethyl, methoxy or ethoxy,
R¹¹ furthermore represents respectively optionally fluorine-, chlorine- or bromine-substituted propenyl or propinyl, and
R¹² represents hydrogen or represents optionally cyano-, fluorine-, chlorine-, methoxy- or ethoxy-substituted methyl, ethyl, n- or i-propyl, n-, i-, s- or t-butyl, n-, i-, s- or t-pentyl,
R¹² furthermore represents respectively optionally fluorine-, chlorine- or bromine-substituted propenyl, butenyl, pentenyl, propinyl, butinyl or pentinyl, represents respectively optionally cyano-, fluorine-, chlorine-, bromine-, methyl-, ethyl-, methoxycarbonyl- or ethoxycarbonyl-substituted cyclopropyl, cyclobutyl, cyclopentyl, cyclohexyl, cyclopropylmethyl, cyclo-butylmethyl, cyclopentylmethyl or cyclohexylmethyl,
R¹² furthermore represents respectively optionally cyano-, fluorine-, chlorine-, bromine-, methyl-, ethyl-, trifluoromethyl-, methoxy-, ethoxy-, difluoromethoxy-, trifluoromethoxy-, methylthio-, ethylthio-, difluoromethylthio-, trifluoromethylthio-, methoxycarbonyl- or ethoxycarbonyl-substituted phenyl, phenylmethyl, phenylethyl, furyl, furylmethyl, tetrahydrofuryl, tetrahydrofurylmethyl, thienyl, thienylmethyl, tetrahydrothienyl, tetrahydrothienylmethyl, perhydropyranyl, perhydropyranylmethyl, oxazolyl, oxazolylmethyl, thiazolyl, thiazolylmethyl, oxadiazolyl, oxadiazolylmethyl, thiadiazolyl, thiadiazolylmethyl, dioxolanyl, dioxolanylmethyl, pyridinyl, pyridinylmethyl, pyrimidinyl or pyrimidinylmethyl.

3. Process for preparing substituted phenyluracils of the general formula (I) 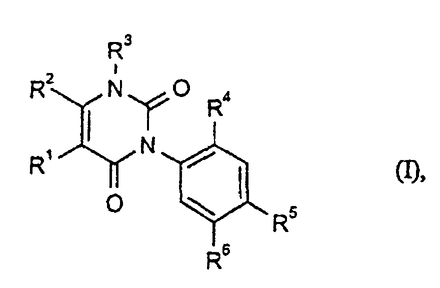 in which
R¹, R², R³, R⁴, R⁵ and R⁶ are each as defined in Claim 1,
**characterized in that**
(a) aminophenyluracils of the general formula (II) 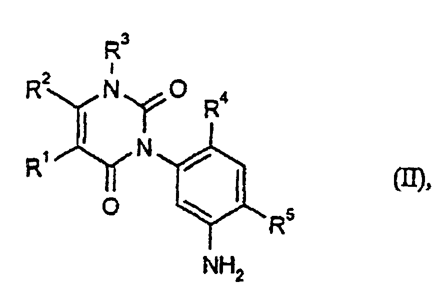 in which
R¹, R², R³, R⁴ and R⁵ are each as defined above,
- or acid adducts of compounds of the formula (II) -
are reacted with an alkali metal nitrite or alkyl nitrite and with a hydrogen halide (HX¹) or a metal halide (MX¹), if appropriate in the presence of a diluent, and the resulting diazonium salts of the general formula (III) 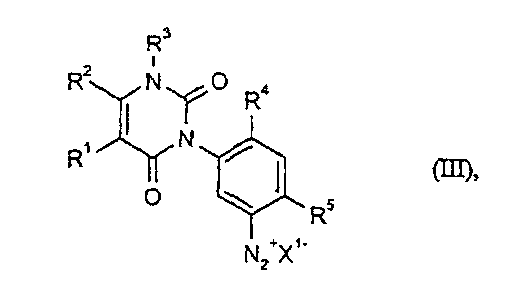 in which
R¹, R², R³, R⁴ and R⁵ are each as defined above and
X¹ represents halogen,
are reacted with acrylic acid derivatives of the general formula (IV)
C(R⁷,R⁸)=C(R⁷)-R⁹ (IV)
in which
R⁷, R⁸ and R⁹ are each as defined above,
in the presence of hydrogen halides (HX¹), if appropriate in the presence of catalysts, if appropriate in the presence of water and if appropriate in the presence of the organic solvent employed initially,
or
(b) substituted phenyluracils of the general formula (Ia) 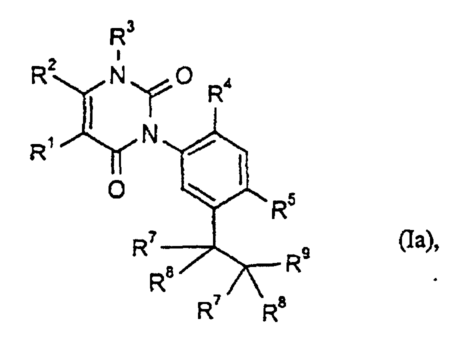 in which
R¹, R², R³, R⁴, R⁵, R⁷, R⁸ and R⁹ are each as defined above,
- but where at least one of the radicals R⁷ / R⁸ represents hydrogen and at least one further radical R⁷ / R⁸ in a position vicinal to the first represents halogen -
are reacted with an acid acceptor, if appropriate in the presence of a diluent.

4. Substituted phenyluracils of the general formula (Ia) 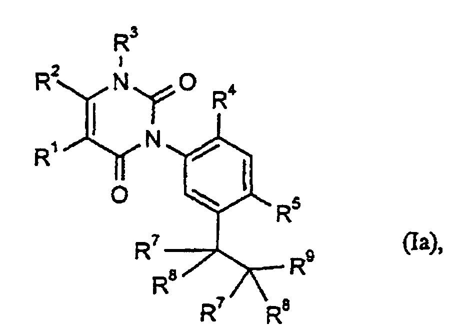 in which
R¹ represents hydrogen, halogen or optionally substituted alkyl,
R² represents optionally substituted alkyl,
R³ represents hydrogen, amino or respectively optionally substituted alkyl, alkenyl or alkinyl,
R⁴ represents hydrogen, cyano or halogen,
R⁵ represents cyano or thiocarbamoyl, and
R⁷ and R⁸ are identical or different and each represents independently of the other hydrogen, hydroxyl, mercapto, halogen or respectively optionally substituted alkyl, alkoxy or alkylthio, and
R⁹ represents cyano, the grouping -CO-OR¹⁰ or the grouping -CO-N(R¹¹,R¹²), where
R¹⁰ represents hydrogen or represents a respectively optionally substituted radical from the group consisting of alkyl, alkenyl, alkinyl, cycloalkyl, cycloalkylalkyl, aryl, arylalkyl, heterocyclyl and heterocyclylalkyl,
R¹¹ represents hydrogen or represents a respectively optionally substituted radical from the group consisting of alkyl, alkoxy, alkenyl and alkinyl, and
R¹² represents hydrogen or represents a respectively optionally substituted radical from the group consisting of alkyl, alkenyl, alkinyl, cycloalkyl, cycloalkylalkyl, aryl, arylalkyl, heterocyclyl and heterocyclylalkyl, or together with R¹¹ represents alkanediyl.

5. Herbicides, **characterized in that** they comprise at least one substituted phenyluracil of the general formula (I) according to Claims 1 to 4.

6. Method for controlling undesirable plants, **characterized in that** substituted phenyluracils of the general formula (I) according to Claims 1 to 4 are allowed to act on undesirable plants and/or their habitat.

7. Use of substituted phenyluracils of the general formula (I) according to Claims 1 to 4 for controlling undesirable plants.

8. Process for preparing herbicides, **characterized in that** substituted phenyluracils of the general formula (I) according to Claims 1 to 4 are mixed with extenders and/or surfactants.

## Revendications

1. Phényluraciles substitués de formule générale (I) 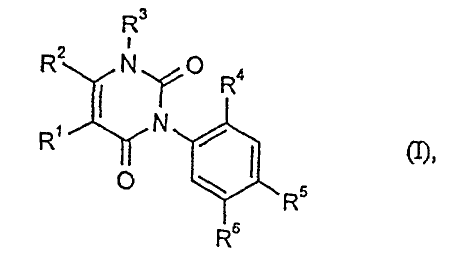 dans laquelle
R¹ représente l'hydrogène, le fluor, le chlore, le brome ou un groupe alkyle en C₁ à C₄ éventuellement substitué par du fluor et/ou par du chlore,
R² représente un groupe alkyle en C₁ à C₄ éventuellement substitué par du fluor et/ou par du chlore,
R³ représente l'hydrogène, un groupe amino, un groupe alkyle en C₁ à C₆ éventuellement substitué par un radical cyano, fluoro, chloro ou alkoxy en C₁ à C₄, ou un groupe alcényle en C₂ à C₆ ou alcynyle en C₂ à C₆ éventuellement substitué par du fluor et/ou par du chlore ;
R⁴ représente l'hydrogène, un groupe cyano, le fluor ou le chlore,
R⁵ représente un groupe cyano ou thiocarbamoyle, et
R⁶ représente l'un des groupements suivants
-C(R⁷,R⁸)-C(R⁷,R⁸)-R⁹ ou -C(R⁷)=C(R⁸)-R⁹
où
R⁷ et R⁸ sont identiques ou différents et représentent, indépendamment l'un de l'autre, l'hydrogène, un groupe hydroxy, mercapto, le fluor, le chlore, le brome, ou bien un groupe alkyle en C₁ à C₄, un groupe alkoxy en C₁ à C₄ ou un groupe alkylthio en C₁ à C₄ dont chacun est éventuellement substitué par un radical cyano, fluoro, chloro ou alkoxy en C₁ à C₄, et
R⁹ représente un groupe cyano, formyle, (alkyle en C₁ à C₄) carbonyle, le groupement -CO-OR¹⁰ ou le groupement -CO-N(R¹¹, R¹²), où
R¹⁰ représente l'hydrogène ou un groupe alkyle en C₁ à C₁₀ éventuellement substitué par un radical cyano, fluoro, chloro ou alkoxy en C₁ à C₄,
R¹⁰ représente en outre un groupe alcényle en C₃ à C₁₀ ou un groupe alcynyle en C₃ à C₁₀ dont chacun est éventuellement substitué par du fluor, du chlore ou du brome,
R¹⁰ représente en outre un groupe cycloalkyle en C₃ à C₆ ou un groupe (cycloalkyle en C₃ à C₆)-(alkyle en C₁ à C₄) dont chacun est éventuellement substitué par un radical cyano, fluoro, chloro, bromo, alkyle en C₁ à C₄ ou (alkoxy en C₁ à C₄)-carbonyle,
R¹⁰ représente en outre un groupe phényle, phényl-(alkyle en C₁ à C₄), furyle, furylméthyle, tétrahydrofuryle, tétrahydrofurylméthyle, thiényle, thiénylméthyle, tétrahydrothiényle, tétrahydrothiénylméthyle, perhydropyrannyle, perhydropyrannylméthyle, oxazolyle, oxazolylméthyle, thiazolyle, thiazolylméthyle, oxadiazolyle, oxadiazolylméthyle, thiadiazolyle, thiadiazolylméthyle, dioxolannyle, dioxolannylméthyle, pyridinyle, pyridinylméthyle, pyrimidinyle ou pyrimidinylméthyle, chacun de ces groupes étant éventuellement substitué par un radical cyano, fluoro, chloro, bromo, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄ ou (alkoxy en C₁ à C₄)-carbonyle,
R¹¹ représente l'hydrogène ou bien un groupe alkyle en C₁ à C₆ ou alkoxy en C₁ à C₆, chacun de ces groupes portant éventuellement un substituant cyano, fluoro, chloro ou alkoxy en C₁ à C₄,
R¹¹ représente en outre un groupe alcényle en C₃ à C₆ ou un groupe alcynyle en C₃ à C₆ dont chacun est éventuellement substitué par du fluor, du chlore ou du brome et
R¹² représente l'hydrogène ou un groupe alkyle en C₁ à C₁₀ éventuellement substitué par un radical cyano, fluoro, chloro ou alkoxy en C₁ à C₄,
R¹² représente en outre un groupe alcényle en C₃ à C₁₀ ou un groupe alcynyle en C₃ à C₁₀, chacun étant éventuellement substitué par du fluor, du chlore ou du brome,
R¹² représente en outre un groupe cycloalkyle en C₃ à C₆ ou (cycloalkyle en C₃ à C₆)-(alkyle en C₁ à C₄), chacun étant éventuellement substitué par un radical cyano, fluoro, chloro, bromo, alkyle en C₁ à C₄ ou alkoxy en C₁ à C₄,
R¹² représente en outre un groupe phényle, phényl-(alkyle en C₁ à C₄), furyle, furylméthyle, tétrahydrofuryle, tétrahydrofurylméthyle, thiényle, thiénylméthyle, tétrahydrothiényle, tétrahydrothiénylméthyle, perhydropyrannyle, perhydropyrannylméthyle, oxazolyle, oxazolylméthyle, thiazolyle, thiazolylméthyle, oxadiazolyle, oxadiazolylméthyle, thiadiazolyle, thiadiazolylméthyle, dioxolannyle, dioxolannylméthyle, pyridinyle, pyridinylméthyle, pyrimidinyle ou pyrimidinylméthyle, chacun étant éventuellement substitué par un radical cyano, fluoro, chloro, bromo, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄ ou (alkoxy en C₁ à C₄)-carbonyle, ou forme conjointement avec R¹¹ un groupe alcanediyle en C₂ à C₆.

2. Phényluraciles substitués de formule générale (I) suivant la revendication 1, **caractérisés en ce que**
R¹ représente l'hydrogène, le fluor, le chlore, le brome ou un groupe méthyle ou éthyle éventuellement substitué par du fluor et/ou par du chlore,
R² représente un groupe méthyle ou éthyle éventuellement substitué par du fluor et/ou par du chlore,
R³ représente l'hydrogène, un groupe amino, un groupe méthyle, éthyle, n-propyle, isopropyle, n-butyle, isobutyle, sec.-butyle ou tertiobutyle portant éventuellement un substituant cyano, fluoro, chloro, méthoxy ou éthoxy, ou un groupe propényle, butényle, propynyle ou butynyle portant éventuellement un substituant fluoro et/ou chloro,
R⁴ représente l'hydrogène, le fluor ou le chlore,
R⁵ représente un groupe cyano ou thiocarbamoyle, et
R⁶ représente l'un des groupements suivants
-C(R⁷,R⁸)-C(R⁷,R⁸)-R⁹ ou -C(R⁷)=C(R⁸)-R⁹
où
R⁷ et R⁸ sont identiques ou différents et représentent indépendamment l'un de l'autre l'hydrogène, le fluor, le chlore, le brome ou un groupe méthyle, éthyle, méthoxy, éthoxy, méthylthio ou éthylthio, chacun de ces groupes étant éventuellement substitué par un radical cyano, fluoro, chloro, méthoxy ou éthoxy, et
R⁹ représente un groupe cyano, le groupement -CO-OR¹⁰ ou le groupement -CO-N(R¹¹, R¹²) où
R¹⁰ représente l'hydrogène ou un groupe méthyle, éthyle, n-propyle, isopropyle, n-butyle, isobutyle, sec.-butyle, tertiobutyle, n-pentyle, isopentyle, sec.-pentyle ou tertiopentyle, portant éventuellement un substituant cyano, fluoro, chloro, méthoxy ou éthoxy,
R¹⁰ représente en outre un groupe propényle, butényle, pentényle, propynyle, butynyle ou pentynyle portant chacun le cas échéant un substituant fluoro, chloro ou bromo,
R¹⁰ représente en outre un groupe cyclopropyle, cyclobutyle, cyclopentyle, cyclohexyle, cyclopropylméthyle, cyclobutylméthyle, cyclopentylméthyle ou cyclohexylméthyle, chacun portant le cas échéant un substituant cyano, fluoro, chloro, bromo, méthyle, éthyle, méthoxycarbonyle ou éthoxycarbonyle,
R¹⁰ représente en outre un groupe phényle, phénylméthyle, phényléthyle, furyle, furylméthyle, tétrahydrofuryle, tétrahydrofurylméthyle, thiényle, thiénylméthyle, tétrahydrothiényle, tétrahydrothiénylméthyle, perhydropyrannyle, perhydropyrannylméthyle, oxazolyle, oxazolylméthyle, thiazolyle, thiazolylméthyle, oxadiazolyle, oxadiazolylméthyle, thiadiazolyle, thiadiazolylméthyle, dioxolannyle, dioxolannylméthyle, pyridinyle, pyridinylméthyle, pyrimidinyle ou pyrimidinylméthyle, chacun étant éventuellement substitué par un radical cyano, fluoro, chloro, bromo, méthyle, éthyle, trifluorométhyle, méthoxy, éthoxy, difluorométhoxy, trifluorométhoxy, méthylthio, éthylthio, difluorométhylthio, trifluorométhylthio, méthoxycarbonyle ou éthoxycarbonyle,
R¹¹ représente l'hydrogène ou un groupe méthyle, éthyle, méthoxy ou éthoxy portant éventuellement dans chaque cas un substituant cyano, fluoro, chloro, méthoxy ou éthoxy,
R¹¹ représente en outre un groupe propényle ou propynyle portant chacun le cas échéant un substituant fluoro, chloro ou bromo,
R¹² représente l'hydrogène ou un groupe méthyle, éthyle, n-propyle, isopropyle, n-butyle, isobutyle, sec.-butyle, tertiobutyle, n-pentyle, isopentyle, sec.-pentyle ou tertiopentyle portant le cas échéant un substituant cyano, fluoro, chloro, méthoxy ou éthoxy,
R¹² représente en outre un groupe propényle, butényle, pentényle, propynyle, butynyle ou pentynyle portant chacun le cas échéant un substituant fluoro, chloro ou bromo, un groupe cyclopropyle, cyclobutyle, cyclopentyle, cyclohexyle, cyclopropylméthyle, cyclobutylméthyle, cyclopentylméthyle ou cyclohexylméthyle portant chacun le cas échéant un substituant cyano, floro, chloro, bromo, méthyle, éthyle, méthoxycarbonyle ou éthoxycarbonyle,
R¹² représente en outre un groupe phényle, phénylméthyle, phényléthyle, furyle, furylméthyle, tétrahydrofuryle, tétrahydrofurylméthyle, thiényle, thiénylméthyle, tétrahydrothiényle, tétrahydrothiénylméthyle, perhydropyrannyle, perhydropyrannylméthyle, oxazolyle, oxadiazolylméthyle, thiazolyle, thiazolylméthyle, oxadiazolyle, oxazolylméthyle, thiadiazolyle, thiadiazolylméthyle, dioxolannyle, dioxolannylméthyle, pyridinyle, pyridinylméthyle, pyrimidinyle ou pyrimidinylméthyle portant chacun le cas échéant un substituant cyano, fluoro, chloro, bromo, méthyle, éthyle, trifluorométhyle, méthoxy, éthoxy, difluorométhoxy, trifluorométhoxy, méthylthio, éthylthio, difluorométhylthio, trifluorométhylthio, méthoxycarbonyle ou éthoxycarbonyle.

3. Procédé de production de phényluraciles substitués de formule générale (I) 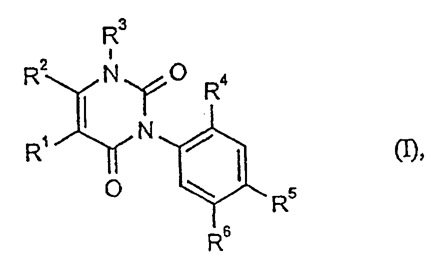 dans laquelle
R¹, R², R³, R⁴, R⁵ et R⁶ ont les définitions indiquées dans la revendication 1,
**caractérisé en ce que**
(a) on fait réagir des aminophényluraciles de formule générale (II) 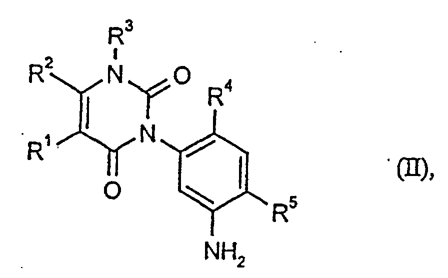 dans laquelle
R¹, R², R³, R⁴ et R⁵ ont les définitions indiquées ci-dessus,
- ou des sels d'addition d'acides de composés de formule (II) -
avec un nitrite de métal alcalin ou d'alkyle et avec un halogénure d'hydrogène (HX¹) ou un halogénure de métal (MX¹), le cas échéant en présence d'un diluant et on fait réagir les sels de diazonium ainsi formés, de formule générale (III) 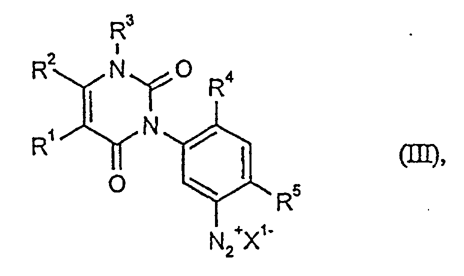 dans laquelle
R¹, R², R³, R⁴ et R⁵ ont les définitions indiquées ci-dessus et
X¹ représente un halogène,
avec des dérivés d'acide acrylique de formule générale (IV)
C(R⁷,R⁸)=C(R⁷)-R⁹ (IV)
dans laquelle
R⁷, R⁸ et R⁹ ont les définitions indiquées ci-dessus,
en présence d'halogénures d'hydrogène (HX¹), le cas échéant en présence de catalyseurs, en présence éventuelle d'eau et
le cas échéant en présence du solvant organique indiqué ci-dessus,
ou bien
(b) on fait réagir des phényluraciles substitués de formule générale (Ia) 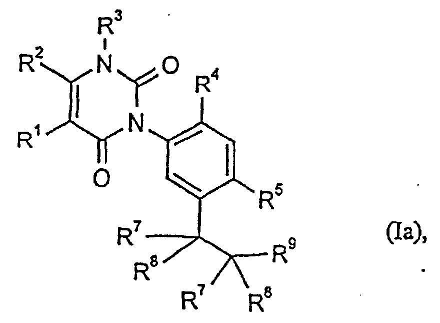 dans laquelle
R¹, R², R³, R⁴, R⁵, R⁷, R⁸ et R⁹ ont les définitions indiquées ci-dessus,
- à la différence qu'au moins l'un des restes R⁷ / R⁸ représente l'hydrogène et qu'au moins un autre des restes R⁷ / R⁸ se trouve dans une position vicinale relative pour un halogène -
avec un accepteur d'acide et le cas échéant en présence d'un diluant.

4. Phényluraciles substitués de formule générale (Ia) 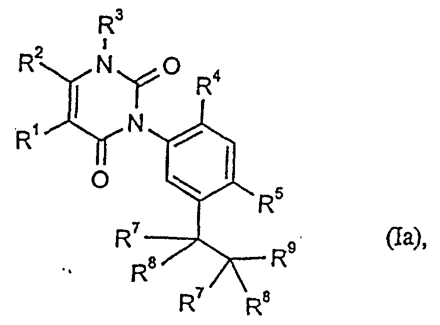 dans laquelle
R¹ représente l'hydrogène, un halogène ou un groupe alkyle éventuellement substitué,
R² est un groupe alkyle éventuellement substitué,
R³ représente l'hydrogène, un groupe amino ou un groupe alkyle, alcényle ou alcynyle dont chacun est éventuellement substitué,
R⁴ est l'hydrogène, un groupe cyano ou un halogène,
R⁵ est un groupe cyano ou thiocarbamoyle, et
R⁷ et R⁸ sont identiques ou différents et représentent chacun, indépendamment l'un de l'autre, l'hydrogène, un groupe hydroxy, mercapto, un halogène ou un groupe alkyle, alkoxy ou alkylthio dont chacun est éventuellement substitué, et
R⁹ représente un groupe cyano, le groupement -CO- OR¹⁰ ou le groupement -CO-N(R¹¹, R¹²), où
R¹⁰ représente l'hydrogène ou un reste, éventuellement substitué dans chaque cas, de la série alkyle, alcényle, alcynyle, cycloalkyle, cycloalkylalkyle, aryle, arylalkyle, hétérocyclyle ou hétérocyclylalkyle,
R¹¹ représente l'hydrogène ou un reste, éventuellement substitué dans chaque cas, de la série alkyle, alkoxy, alcényle ou alcynyle, et
R¹² représente l'hydrogène ou un reste, éventuellement substitué dans chaque cas, de la série alkyle, alcényle, alcynyle, cycloalkyle, cycloalkylalkyle, aryle, arylalkyle, hétérocyclyle ou hétérocyclylalkyle ou forme un groupe alcanediyle conjointement avec R¹¹.

5. Compositions herbicides, **caractérisées par** une teneur en au moins un phényluracile substitué de formule générale (I) suivant les revendications 1 à 4 .

6. Procédé pour combattre des plantes indésirables, **caractérisé en ce qu'**on fait agir des phényluraciles substitués de formule générale (I) suivant les revendications 1 à 4 sur des plantes indésirables et/ou sur leur milieu.

7. Utilisation de phényluraciles substitués de formule générale (I) suivant les revendications 1 à 4 pour combattre des plantes indésirables.

8. Procédé de préparation de compositions herbicides, **caractérisé en ce qu'**on mélange des phényluraciles substitués de formule générale (I) suivant les revendications 1 à 4 avec des diluants et/ou des agents tensioactifs.
